(19) Europäisches Patentamt
European Patent Office
Office européen des brevets

(11) **EP 4 170 662 A1**

(12) **EUROPEAN PATENT APPLICATION**
published in accordance with Art. 153(4) EPC

(43) Date of publication:
**26.04.2023  Bulletin 2023/17**

(21) Application number: **21879216.6**

(22) Date of filing: **22.09.2021**

(51) International Patent Classification (IPC):
**G16C 20/50** (2019.01)

(86) International application number:
**PCT/CN2021/119651**

(87) International publication number:
**WO 2022/078170 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.10.2020  CN 202011112368**

(71) Applicant: **Tencent Technology (Shenzhen) Company Limited**
**Shenzhen, Guangdong 518057 (CN)**

(72) Inventors:
• **XU, Tingyang**
  **Shenzhen, Guangdong 518057 (CN)**
• **ZHANG, Jiying**
  **Shenzhen, Guangdong 518057 (CN)**
• **YE, Fei**
  **Shenzhen, Guangdong 518057 (CN)**
• **BIAN, Yatao**
  **Shenzhen, Guangdong 518057 (CN)**
• **RONG, Yu**
  **Shenzhen, Guangdong 518057 (CN)**

(74) Representative: **Gunzelmann, Rainer**
**Wuesthoff & Wuesthoff**
**Patentanwälte PartG mbB**
**Schweigerstraße 2**
**81541 München (DE)**

(54) **METHODS FOR DETERMINING INTERACTION INFORMATION AND FOR TRAINING PREDICTION MODEL, AN APPARATUS, AND MEDIUM**

(57)    A method for determining interaction information, an interaction information prediction model training method, a device, and a medium are provided. The interaction information determining method includes: obtaining (201) basic information of a first target object, basic information of a second target object, and a target interaction information prediction model, the target interaction information prediction model being trained by using a global-level loss function and a key local-level loss function; and invoking (202) the target interaction information prediction model to process the basic information of the first target object and the basic information of the second target object, and obtaining target interaction information between the first target object and the second target object. Based on the foregoing process, the process of training the interaction information prediction model not only pays attention to the global information, but also pays attention to the key local information, so that the effect of training the interaction information prediction model is relatively good, and the accuracy of the determined interaction information between the first target object and the second target object is relatively high.

FIG. 2

EP 4 170 662 A1

**Description**

RELATED APPLICATION

This application claims priority to Chinese Patent Application No. 202011112368.9, entitled " METHOD AND APPARATUS FOR DETERMINING INTERACTION INFORMATION, DEVICE, AND STORAGE MEDIUM" filed on October 16, 2020., which is incorporated herein by reference in its entirety.

FIELD OF THE TECHNOLOGY

Embodiments of this application relate to the field of artificial intelligence technologies, and in particular, to a method for determining interaction information, a device, and a medium.

BACKGROUND OF THE DISCLOSURE

With the quick development of artificial intelligence technologies, there are more and more application scenarios in which an interaction information prediction model is used for determining interaction information between two target objects. For example, the interaction information prediction model is used for determining a pIC50 value between a target (that is, protein) and a drug (e.g. a small molecule of a drug) as interaction information, to screen the drug. The foregoing pIC50 value is used for representing a negative logarithm of the concentration of the small molecule achieving an inhibitory effect of 50% on the protein.

In the related art, only a global-level loss function is used for training the interaction information prediction model, and then an interaction information prediction model obtained through training is used for determining interaction information between two target objects. In such a process, the global-level loss function can only cause the process of training the interaction information prediction model to pay attention to global information. As a result, the information to which the attention is paid is relatively limited, the effect of training the interaction information prediction model is not good, and the accuracy of the interaction information between the two target objects determined by using the trained interaction information prediction model is relatively low.

SUMMARY

Embodiments of this application provide a method for determining interaction information, a device, and a medium, which may be used for improving the effect of training an interaction information prediction model and improving the accuracy of interaction information between two target objects determined by using a trained interaction information prediction model. The technical solutions are as follows:

According to an aspect, an embodiment of this application provides a method for determining interaction information, the method including:

obtaining basic information of a first target object, basic information of a second target object, and a target interaction information prediction model, the first target object and the second target object each comprising a protein or a small molecule of a drug respectively, the target interaction information prediction model being trained by using a global-level loss function and a key local-level loss function, the key local-level loss function being determined based on attention information corresponding to one or more key sub-sample objects in one or more sample objects meeting a reference condition, and the one or more key sub-sample objects in the one or more sample objects meeting the reference condition being a part of all sub-sample objects of the one or more sample objects meeting the reference condition, one key sub-sample object in one sample object being a sub-sample object interacting with another sample object, one sample object meeting the reference condition indicating the one sample object comprises at least one key sub-sample object; and

obtaining target interaction information for drug screening between the first target object and the second target object, by invoking the target interaction information prediction model to process the basic information of the first target object and the basic information of the second target object.

An interaction information prediction model training method is further provided, the method including:

obtaining attention information corresponding to a first sample object, attention information corresponding to a second sample object, and predictive interaction information between the first sample object and the second sample object by invoking an initial interaction information prediction model, the predictive interaction information being obtained based on global feature information corresponding to the first sample object and global feature information corresponding to the second sample object;

obtaining, based on the predictive interaction information and standard interaction information between the first sample object and the second sample object, a global-level loss function;

determining, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object;

obtaining, based on the attention information corresponding to the one or more key sub-sample objects in the one or more sample objects meeting the reference condition, a key local-level loss function;

reversely updating, based on the global-level loss function and the key local-level loss function, a parameter of the initial interaction information prediction model; and

obtaining, in response to a parameter update process meeting a termination condition, a target interaction information prediction model.

According to another aspect, a apparatus for determining interaction information is provided, the apparatus including:

an obtaining unit, configured to obtain basic information of a first target object, basic information of a second target object, and a target interaction information prediction model, the first target object and the second target object each comprising a protein or a small molecule of a drug respectively, the target interaction information prediction model being trained by using a global-level loss function and a key local-level loss function, the key local-level loss function being determined based on attention information corresponding to one or more key sub-sample objects in one or more sample objects meeting a reference condition, and the one or more key sub-sample objects in the one or more sample objects meeting the reference condition being a part of all sub-sample objects of the one or more sample objects meeting the reference condition, one key sub-sample object in one sample object being a sub-sample object interacting with another sample object, one sample object meeting the reference condition indicating the one sample object comprises at least one key sub-sample object; and

a processing unit, configured to obtain target interaction information for drug screening between the first target object and the second target object by invoking the target interaction information prediction model to process the basic information of the first target object and the basic information of the second target object.

An interaction information prediction model training apparatus is further provided, the apparatus including:

a first obtaining unit, configured to obtain attention information corresponding to a first sample object, attention information corresponding to a second sample object, and predictive interaction information between the first sample object and the second sample object by invoking an initial interaction information prediction model, the predictive interaction information being obtained based on global feature information corresponding to the first sample object and global feature information corresponding to the second sample object;

a second obtaining unit, configured to obtain, based on the predictive interaction information and standard interaction information between the first sample object and the second sample object, a global-level loss function;

a determining unit, configured to determine, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object;

a third obtaining unit, configured to obtain, based on the attention information corresponding to the one or more key sub-sample objects in the one or more sample objects meeting the reference condition, a key local-level loss function;

an update unit, configured to reversely update, based on the global-level loss function and the key local-level loss function, a parameter of the initial interaction information prediction model; and

a fourth obtaining unit, configured to obtain, in response to a parameter update process meeting a termination condition, a target interaction information prediction model.

According to another aspect, a computer device is provided, the computer device including a processor and a memory, the memory storing at least one piece of program code, the at least one piece of program code being loaded and executed by the processor to cause the computer device to implement the interaction information determining method or the interaction information prediction model training method described above.

According to another aspect, a non-transitory computer-readable storage medium is further provided, the non-transitory computer-readable storage medium storing at least one piece of program code, the at least one piece of program code being loaded and executed by a processor to cause a computer to implement the interaction information determining method or the interaction information prediction model training method described above.

According to another aspect, a computer program product or a computer program is further provided, the computer program product or the computer program including computer instructions, the computer instructions being stored in a computer-readable storage medium. A processor of a computer device reads the computer instructions from the computer-readable storage medium, and the processor executes the computer instructions to cause the computer device to implement the interaction information determining method or the interaction information prediction model training method described above.

The technical solutions provided in the embodiments of this application may bring the following beneficial effects:

In the embodiments of this application, the global-level loss function and the key local-level loss function are used for training the interaction information prediction model, and then the trained interaction information prediction model is used for determining the interaction information between the first target object and the second target object. The global-level loss function can cause the model training process to pay attention to the global information; and the key local-level loss function can cause the model training process to pay attention to the key local information. That is to say, in the embodiments of this application, the process of training the interaction information prediction model not only pays attention to the global information, but also pays attention to the key local information, so that the effect of training the interaction information prediction model is relatively good, and the accuracy of the interaction information between the first target object and the second target object determined by using the trained interaction information prediction model is relatively high.

BRIEF DESCRIPTION OF THE DRAWINGS

FIG. 1 is a schematic diagram of an implementation environment of a method for determining interaction information and an interaction information prediction model training method according to embodiments of this application.

FIG. 2 is a flowchart of a method for determining interaction information according to an embodiment of this application.

FIG. 3 is a schematic diagram of partial graph information of a first target object according to an embodiment of this application.

FIG. 4 is a flowchart of a method for obtaining target interaction information between a first target object and a second target object according to an embodiment of this application.

FIG. 5 is a schematic diagram of a process of obtaining target interaction information between a first target object and a second target object according to an embodiment of this application.

FIG. 6 is a flowchart of an interaction information prediction model training method according to an embodiment of this application.

FIG. 7 is a schematic diagram of an interaction information determining apparatus according to an embodiment of this application.

FIG. 8 is a schematic structural diagram of a processing unit according to an embodiment of this application.

FIG. 9 is a schematic diagram of a apparatus for determining interaction information according to an embodiment of this application.

FIG. 10 is a schematic diagram of an interaction information prediction model training apparatus according to an embodiment of this application.

FIG. 11 is a schematic structural diagram of a computer device according to an embodiment of this application.

DESCRIPTION OF EMBODIMENTS

To make the objectives, technical solutions, and advantages of this application clearer, the following further describes the implementations of this application in detail with reference to the accompanying drawings.

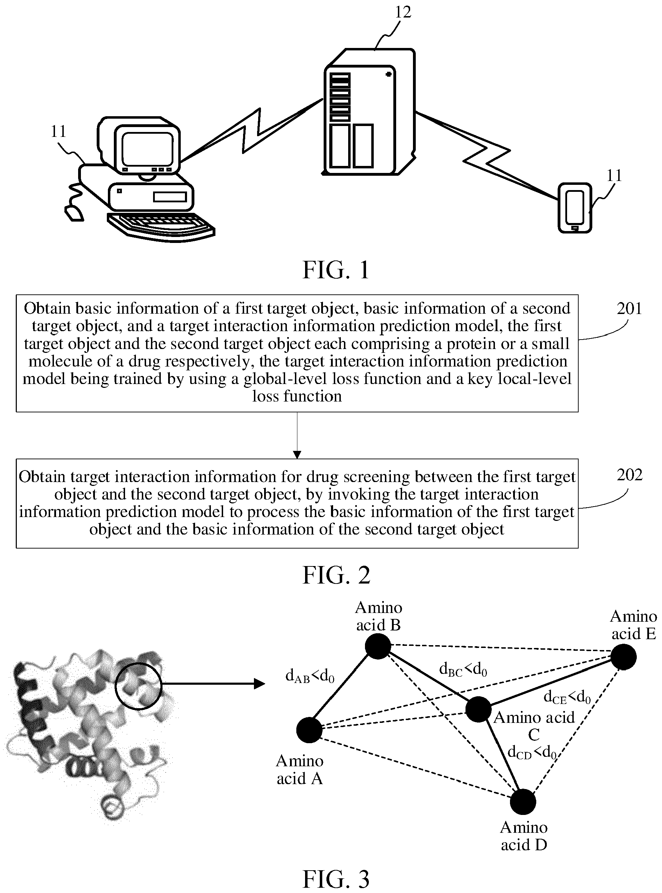

Embodiments of this application provide an interaction information determining method and an interaction information prediction model training method. FIG. 1 is a schematic diagram of an implementation environment of the interaction information determining method and the interaction information prediction model training method according to the embodiments of this application. The implementation environment includes: a terminal 11 and a server 12.

The interaction information determining method provided in the embodiments of this application may be performed by the terminal 11, or may be performed by the server 12. This is not limited in the embodiments of this application. In an exemplary embodiment, for a case that the interaction information determining method provided in the embodiments of this application is performed by the terminal 11, the terminal 11 can send obtained interaction information to the server 12 for storage, and certainly the terminal 11 can also store the obtained interaction information.

In an exemplary embodiment, for a case that the interaction information determining method provided in the embodiments of this application is performed by the server 12, the server 12 can send obtained interaction information to the terminal 11 for storage, and certainly the server 12 can also store the obtained interaction information.

The interaction information prediction model training method provided in the embodiments of this application may be performed by the terminal 11, or may be performed by the server 12. This is not limited in the embodiments of this application. In an exemplary embodiment, for a case that the interaction information prediction model training method provided in the embodiments of this application is performed by the terminal 11, the terminal 11 can send a target interaction information prediction model obtained through training to the server 12 for storage, and certainly the terminal 11 can also store the target interaction information prediction model obtained through training. In an exemplary embodiment, for a case that the interaction information prediction model training method provided in the embodiments of this application is performed by the server 12, the server 12 can send a target interaction information prediction model obtained through training to the terminal 11 for storage, and certainly the server 12 can also store the target interaction information prediction model obtained through training.

In a possible implementation, the terminal 11 may be a smart device such as a mobile phone, a tablet computer, a personal computer, or the like. The server 12 may be one server, a server cluster including a plurality of servers, or a cloud computing service center. The terminal 11 and the server 12 establish a communication connection through a wired or wireless network.

A person skilled in the art is to understand that the terminal 11 and server 12 are only examples, and other existing or potential terminals or servers that are applicable to this application are also to be included in the scope of protection of this application, and are included herein by reference.

Based on the foregoing implementation environment shown in FIG. 1, an embodiment of this application provides a method for determining interaction information, the method for determining interaction information is performed by a computer device, and the computer device may be the terminal 11, or may be the server 12. In this embodiment of this application, a description is made using an example in which the interaction information determining method is applied to the server 12. As shown in FIG. 2, the interaction information determining method provided in this embodiment of this application may include the following step 201 and step 202:

Step 201. Obtain basic information of a first target object, basic information of a second target object, and a target interaction information prediction model, the first target object and the second target object each comprising a protein or a small molecule of a drug respectively, the target interaction information prediction model being trained by using a global-level loss function and a key local-level loss function.

The key local-level loss function is determined based on attention information corresponding to one or more key sub-sample objects in one or more sample objects meeting a reference condition, and the one or more key sub-sample objects in the one or more sample objects meeting the reference condition are a part of all sub-sample objects of the one or more sample objects meeting the reference condition, one key sub-sample object in one sample object is a sub-sample object interacting with another sample object, one sample object meeting the reference condition indicates the one sample object comprises at least one key sub-sample object.

The first target object and the second target object refer to two target objects whose interaction information needs to be determined by using the target interaction information prediction model. In this embodiment of this application, types of the first target object and the second target object are not limited. Exemplarily, the type of each of the first target object and the second target object is a protein; or the type of each of the first target object and the second target object is a small molecule; or, the type of one target object of the first target object and the second target object is a protein, and the type of the other target object is a small molecule. Each of the first target object and the second target object includes at least one sub-target object. When the type of a target object is a protein, the type of a sub-target object included in the target object is an amino acid; and when the type of a target object is a small molecule, the type of a sub-target object included in the target object is an atom.

The protein has a spatial structure, and the protein is formed by folding a chain of amino acids in space; and the small molecule refers to a molecule whose molecular weight is relatively small. Exemplarily, the small molecule is a molecule whose molecular weight is less than 500. In an exemplary embodiment, the small molecule refers to a drug. For a case that the type of one target object of the first target object and the second target object is a protein and the type of the other target object is a small molecule, a process of determining interaction information between the two target objects is used for virtually screening drugs. Virtual screening plays a quite important role in drug development, and may greatly reduce time and expense required by a related experiment. In this embodiment of this application, the target interaction information prediction model obtained through training can be used for performing virtual screening of drugs, and feature information of the protein and the small molecule is effectively used, thereby improving reliability of virtual screening of drugs.

The basic information of the first target object is information that is used for being inputted to the target interaction information prediction model and that is used for representing the first target object. The basic information of the second target object is information that is used for being inputted to the target interaction information prediction model and that is used for representing the second target object. The basic information of the first target object and the basic information of the second target object provide data support for the process of predicting the target interaction information prediction model. In an exemplary embodiment, the basic information of the first target object is graph information of the first target object; and the basic information of the second target object is graph information of the second target object. Graph information of a target object is used for representing the target object in the form of a graph including nodes and edges.

In a possible implementation, an implementation of obtaining the basic information of the first target object and the basic information of the second target object is related to the type of the first target object and the type of the second target object. In an exemplary embodiment, the type of the first target object and the type of the second target object include the following four cases:

Case 1: The type of the first target object is a protein, and the type of the second target object is a small molecule.

In a possible implementation, in this case 1, the implementation of obtaining the basic information of the first target object and the basic information of the second target object includes the following step 1 to step 4:

Step 1: Determine, based on structural information of the first target object, a spatial distance between amino acids in the first target object.

When the type of the first target object is a protein, the structural information of the first target object refers to structural information of the protein corresponding to the first target object, the structural information of the protein is recorded in a structure file of the protein, and the structural information of the first target object can be obtained in the structure file of the protein corresponding to first target object.

When the type of the first target object is a protein, the type of a sub-target object included in the first target object is an amino acid. Structural information of the first target object includes basic information of each amino acid in the first target object, and a spatial distance between amino acids in the first target object may be determined according to the basic information of each amino acid. The spatial distance between the amino acids in the first target object includes a spatial distance between any two amino acids in the first target object.

In a possible implementation, after the spatial distance between amino acids in the first target object is determined, the spatial distance between amino acids may be further standardized, to obtain a standard spatial distance between amino acids. The standard spatial distance between any two amino acids can provide a reference for obtaining a basic feature of an edge connecting nodes corresponding to the any two amino acids.

In an exemplary embodiment, for an amino acid i and an amino acid j in the protein corresponding to the first target object, a standard spatial distance between the amino acid i and the amino acid j is obtained based on a formula 1:

$$\hat{s}_{ij} = \frac{1}{1 + d_{ij}/d'} \quad \text{(formula 1)}$$

where $\hat{s}_{ij}$ represents the standard spatial distance between the amino acid i and the amino acid j; $d_{ij}$ represents a spatial

distance between the amino acid i and the amino acid j; $d'$ represents a scaling dimension, and exemplarily, a value of $d'$ is 3.8 Å.

Step 2: Determine, based on the spatial distance between the amino acids, an adjacency matrix corresponding to the first target object, the adjacency matrix being used for indicating an association between the amino acids in the first target object.

After the spatial distance between amino acids in the first target object is determined, the adjacency matrix used for indicating the association between the amino acids in the first target object is determined based on the spatial distance between amino acids. In a possible implementation, the adjacency matrix is formed by a value used for indicating an association between any two amino acids. Exemplarily, a value used for indicating an association between the amino acid i and the amino acid j is determined based on a formula 2:

$$A_{ij} = \begin{cases} 1, & d_{ij} < d_0 \\ 0, & d_{ij} > d_0 \end{cases} \quad \text{(formula 2)}$$

where $A_{ij}$ represents the value used for indicating an association between the amino acid i and the amino acid j, where if $A_{ij}$ is 1, it indicates that an association exists between the amino acid i and the amino acid j, and if $A_{ij}$ is 0, it indicates that no association exists between the amino acid i and the amino acid j; $d_{ij}$ represents a spatial distance between the amino acid i and the amino acid j; $d_0$ represents a distance threshold, and exemplarily, a value of $d_0$ is 12 Å. The value used for indicating an association between any two amino acids can be determined based on the formula 2, thereby obtaining the adjacency matrix corresponding to the first target object, and whether an association exists between any two amino acids in the first target object can be determined according to the adjacency matrix corresponding to the first target object.

Step 3: Obtain the graph information of the first target object according to the adjacency matrix corresponding to the first target object and the amino acids in the first target object, and use the graph information of the first target object as the basic information of the first target object.

Graph information of the first target object is used for representing the first target object in the form of a graph. In a possible implementation, the process of obtaining the graph information of the first target object according to the adjacency matrix corresponding to the first target object and the amino acids in the first target object is: using the amino acids in the first target object as nodes, constructing edges between the nodes according to the adjacency matrix, and obtaining the graph information of the first target object.

In a possible implementation, the process of constructing edges between the nodes according to the adjacency matrix is: for two nodes corresponding to any two amino acids, constructing an edge between the two nodes corresponding to the any two amino acids if it is determined based on the adjacency matrix that an association exists between the any two amino acids (for example, a value used for indicating the association between the any two amino acids in the adjacency matrix is 1); and constructing no edge between the two nodes corresponding to the any two amino acids if it is determined based on the adjacency matrix that no association exists between the any two amino acids (for example, a value used for indicating the association between the any two amino acids in the adjacency matrix is 0).

Exemplarily, partial graph information of the first target object is shown in FIG. 3. In the partial graph information shown in FIG. 3, each of an amino acid A, an amino acid B, an amino acid C, an amino acid D, and an amino acid E is a node, and because a spatial distance between the amino acid A and the amino acid B is less than the distance threshold (that is, $d_{AB} < d_0$), an edge exists between two nodes corresponding to the amino acid A and the amino acid B. Similarly, an edge exists between two nodes corresponding to the amino acid B and the amino acid C, between two nodes corresponding to the amino acid C and the amino acid D, and between two nodes corresponding to the amino acid C and the amino acid E.

After the graph information of the first target object is obtained, the graph information of the first target object is used as the basic information of the first target object, thereby obtaining the basic information of the first target object.

Step 4: Obtain, based on atoms in the second target object and chemical bond information between the atoms, the graph information of the second target object, and use the graph information of the second target object as the basic information of the second target object.

Graph information of the second target object is used for representing the second target object in the form of a graph. When the type of the second target object is a small molecule, the type of a sub-target object included in the second target object is an atom. In a possible implementation, the process of obtaining, based on atoms in the second target object and chemical bond information between the atoms, the graph information of the second target object is: using the atoms in the second target object as nodes, constructing edges between the nodes according to the chemical bond information between the atoms, and obtaining the graph information of the second target object.

In an exemplary embodiment, the process of constructing edges between the nodes according to the chemical bond information between the atoms is: for any two atoms, constructing an edge between nodes corresponding to the any two atoms if chemical bond information between the atoms indicates that a chemical bond connection exists between the any two atoms; and constructing no edge between the nodes corresponding to the any two atoms if the chemical bond information between the atoms indicates that no chemical bond connection exists between the any two atoms.

After the graph information of the second target object is obtained, the graph information of the second target object is used as the basic information of the second target object, thereby obtaining the basic information of the second target object.

Case 2: The type of the first target object is a small molecule, and the type of the second target object is a protein.

In a possible implementation, in this case 2, the implementation of obtaining the basic information of the first target object is: obtaining, based on atoms in the first target object and chemical bond information between the atoms, the graph information of the first target object, and using the graph information of the first target object as the basic information of the first target object. For the implementation of obtaining the basic information of the first target object, reference is made to step 4 in the case 1, and details are not described herein again.

The implementation of obtaining the basic information of the second target object is: determining, based on structural information of the second target object, a spatial distance between amino acids in the second target object; determining, based on the spatial distance between the amino acids, an adjacency matrix corresponding to the second target object, the adjacency matrix being used for indicating an association between the amino acids in the second target object; and obtaining the graph information of the second target object according to the adjacency matrix corresponding to the second target object and the amino acids in the second target object, and using the graph information of the second target object as the basic information of the second target object. For the implementation of obtaining the basic information of the second target object, reference is made to step 1 to step 3 in the case 1, and details are not described herein again.

Case 3: The type of the first target object is a protein, and the type of the second target object is a protein.

In a possible implementation, in this case 3, the implementation of obtaining the basic information of the first target object is: determining, based on structural information of the first target object, a spatial distance between amino acids in the first target object; determining, based on the spatial distance between the amino acids, an adjacency matrix corresponding to the first target object, the adjacency matrix being used for indicating an association between the amino acids in the first target object; and obtaining the graph information of the first target object according to the adjacency matrix corresponding to the first target object and the amino acids in the first target object, and using the graph information of the first target object as the basic information of the first target object.

The implementation of obtaining the basic information of the second target object is: determining, based on structural information of the second target object, a spatial distance between amino acids in the second target object; determining, based on the spatial distance between the amino acids, an adjacency matrix corresponding to the second target object, the adjacency matrix being used for indicating an association between the amino acids in the second target object; and obtaining the graph information of the second target object according to the adjacency matrix corresponding to the second target object and the amino acids in the second target object, and using the graph information of the second target object as the basic information of the second target object.

For each of the implementation of obtaining the basic information of the first target object and the implementation of obtaining the basic information of the second target object, reference is made to step 1 to step 3 in the case 1, and details are not described herein again.

Case 4: The type of the first target object is a small molecule, and the type of the second target object is a small molecule.

In a possible implementation, in this case 4, the implementation of obtaining the basic information of the first target object is: obtaining, based on atoms in the first target object and chemical bond information between the atoms, the graph information of the first target object, and using the graph information of the first target object as the basic information of the first target object. The implementation of obtaining the basic information of the second target object is: obtaining, based on atoms in the second target object and chemical bond information between the atoms, the graph information of the second target object, and using the graph information of the second target object as the basic information of the second target object. For each of the implementation of obtaining the basic information of the first target object and the implementation of obtaining the basic information of the second target object, reference is made to step 4 in the case 1, and details are not described herein again.

The above description is only an exemplary description of obtaining the basic information of the first target object and the basic information of the second target object, and this embodiment of this application is not limited thereto. In an exemplary embodiment, the basic information of the first target object and the basic information of the second target object may alternatively be other information different from the graph information, as long as the information can provide data support for the process of predicting the target interaction information prediction model. This is not limited in this embodiment of this application.

In a possible implementation, after the basic information of the first target object and the basic information of the second target object are obtained, the basic information of the first target object and the basic information of the second target object may be stored, to make it convenient to obtain the basic information of the first target object and the basic information of the second target object subsequently in a direct extracting manner.

To determine the interaction information between the first target object and the second target object, in addition to obtaining the basic information of the first target object and the basic information of the second target object, the target interaction information prediction model further needs to be obtained. The target interaction information prediction model refers to a trained interaction information prediction model. The target interaction information prediction model is trained by using a global-level loss function and a key local-level loss function. The global-level loss function is used for causing the model training process to pay attention to the global information; and the key local-level loss function is used for causing the model training process to pay attention to the key local information. That is to say, the process of obtaining the target interaction information prediction model through training not only pays attention to the global information, but also pays attention to the key local information, and the training effect is relatively good.

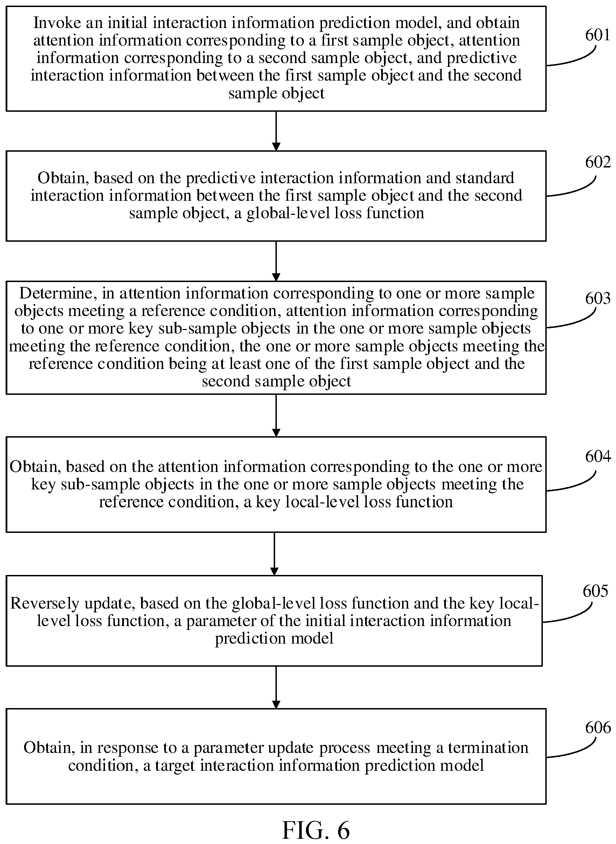

The process of obtaining the target interaction information prediction model through training will be described in detail in an embodiment shown in FIG. 6, but is not described in detail now. That is, related content of the global-level loss function and the key local-level loss function will be described in detail in the embodiment shown in FIG. 6, but is not described in detail now.

In step 201, the manner of obtaining the target interaction information prediction model may be directly extracting the trained target interaction information prediction model, or may be obtaining the target interaction information prediction model through training. This is not limited in this embodiment of this application. For the case of directly extracting the trained target interaction information prediction model, the process of obtaining the target interaction information prediction model through training has been completed before step 201 is performed, and the target interaction information prediction model obtained through training is stored.

Step 202. Obtain target interaction information for drug screening between the first target object and the second target object, by invoking the target interaction information prediction model to process the basic information of the first target object and the basic information of the second target object.

The target interaction information between the first target object and the second target object is information used for reflecting interaction between the first target object and the second target object and predicted by using the target interaction information prediction model. The meaning of the target interaction information between the first target object and the second target object is related to the types of the first target object and the second target object. In an exemplary embodiment, when each of the first target object and the second target object is a small molecule, the interaction information between the first target object and the second target object indicates chemical reaction information between the two small molecules; when each of the first target object and the second target object is a protein, the interaction information between the first target object and the second target object indicates binding information between the two proteins; and when one target object of the first target object and the second target object is a protein, and the other target object is a small molecule, the interaction information between the first target object and the second target object indicates protein-small molecule active information, and the protein-small molecule active information can screen the small molecule.

The representation form of the protein-small molecule active information is not limited in this embodiment of this application. Exemplarily, the protein-small molecule active information is represented using a pIC50 value, where $pIC50 = -\lg(IC50)$, IC50 is used for representing the concentration of the small molecule achieving an inhibitory effect of 50% on the protein.

After the basic information of the first target object, the basic information of the second target object, and the target interaction information prediction model are obtained, the target interaction information prediction model may be invoked to process the basic information of the first target object and the basic information of the second target object, thereby obtaining the target interaction information between the first target object and the second target object.

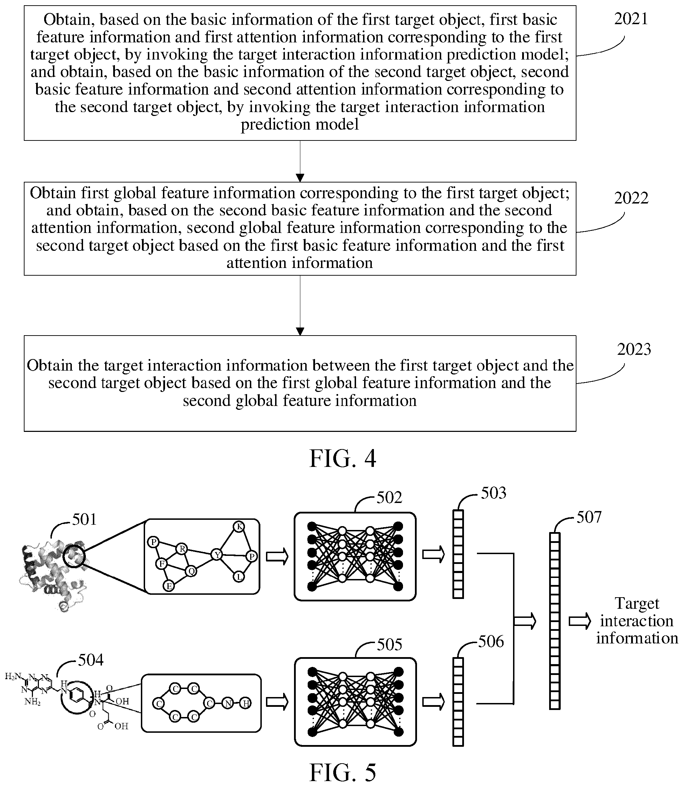

In a possible implementation, referring to FIG. 4, a process of implementing step 202 includes the following step 2021 to step 2023:

Step 2021: Obtain, based on the basic information of the first target object, first basic feature information and first attention information corresponding to the first target object, by invoking the target interaction information prediction model; and obtain, based on the basic information of the second target object, second basic feature information and second attention information corresponding to the second target object, by invoking the target interaction information prediction model.

In a possible implementation, the process of obtaining, based on the basic information of the first target object, first basic feature information and first attention information corresponding to the first target object includes the following step 2021a and step 2021b:

Step 2021a: Obtain, based on the basic information of the first target object, first basic feature information corresponding to the first target object.

In a possible implementation, the target interaction information prediction model includes a first feature extraction model, and the first feature extraction model is used for extracting the first basic feature information corresponding to the first target object based on the basic information of the first target object. Based on this, the process of obtaining, based on the basic information of the first target object, first basic feature information corresponding to the first target object is: invoking the first feature extraction model to perform feature extraction on the basic information of the first target object, and obtaining the first basic feature information corresponding to the first target object. The model structure of the first feature extraction model is not limited in this embodiment of this application, as long as the model structure can determine the first basic feature information corresponding to the first target object based on the basic information of the first target object. In an exemplary embodiment, when the basic information of the first target object is the graph information of the first target object, the model structure of the first feature extraction model is a dual-message passing model formed by a node message passing model and an edge message passing model.

In a possible implementation, the basic information of the first target object is the graph information of the first target object, the first feature extraction model includes a first node message passing model and a first edge message passing model, and the first basic feature information includes node-level first basic feature information and edge-level first basic feature information. In this case, the process of obtaining the first basic feature information corresponding to the first target object by invoking the first feature extraction model to perform feature extraction on the basic information of the first target object includes: obtaining the node-level first basic feature information by invoking the first node message passing model to perform node-level feature extraction on the graph information of the first target object; and obtaining the edge-level first basic feature information by invoking the first edge message passing model to perform edge-level feature extraction on the graph information of the first target object. In an exemplary embodiment, the model structure of each of the first node message passing model and the first edge message passing model is a message passing neural network (MPNN).

In a possible implementation, the first node message passing model includes a first node feature update layer and a first node-level feature output layer, and the process of obtaining the node-level first basic feature information by invoking the first node message passing model to perform node-level feature extraction on the graph information of the first target object includes the following step A and step B:

Step A: Obtain a target node feature by invoking the first node feature update layer to update a node feature in the graph information of the first target object.

The target node feature includes an updated node feature of each node in the graph information of the first target object obtained after processing through the first node feature update layer. The first node feature update layer in the first node message passing model aggregates features of nodes around a node and features of all edges to the node, to obtain an updated node feature of the node through update of a reference quantity of steps. The reference quantity is set according to experience, or flexibly adjusted according to an application scenario. This is not limited in this embodiment of this application. A node in the graph information of the first target object corresponds to a sub-target object in the first target object. If the type of the first target object is a protein, a node in the graph information of the first target object corresponds to an amino acid. If the type of the first target object is a small molecule, a node in the graph information of the first target object corresponds to an atom.

In an exemplary embodiment, the process of invoking the first node feature update layer to update a node feature in the graph information of the first target object is implemented based on a formula 3:

$$h_v^0 = \sigma\left(W_{in}x_v\right)$$
$$m_v^{d+1} = \sum_{k \in N(v)} cat\left(h_k^d, e_{vk}\right)$$
$$h_v^{d+1} = \sigma\left(W_a m_v^{d+1} + h_v^0\right) \quad \text{(formula 3)}$$

where $h_v^0$ represents an initial node feature of a node $v$; $\sigma(\cdot)$ represents an activation function; $x_v$ represents a basic feature of a node $v$, and the basic feature of the node $v$ is determined based on basic information of a sub-target object corresponding to the node $v$. Exemplarily, when the type of the first target object is a protein, the basic feature of the node $v$ is determined based on basic information of an amino acid corresponding to the node $v$, and the basic information of the amino acid includes but not limited to a type of the amino acid, a sequence number of the amino acid, a structure of the amino acid, and the like. When the type of the first target object is a small molecule, the basic feature of the node $v$ is determined based on basic information of an atom corresponding to the node $v$, and the basic information of the atom includes but not limited to a type of the atom, a sequence number of the atom, a charge number of the atom, and

the like. $m_v^{d+1}$ represents a value of a message passing function of a $(d+1)^{th}$ (d is an integer not less than 0) step corresponding to the node $v$; $cat(\cdot,\cdot)$ represents a concatenating function; $N(v)$ represents a set of adjacent nodes of the node $v$; $h_k^d$ represents an updated node feature of a node $k$ through $d$ steps; $e_{vk}$ represents a basic feature of an edge from the node $k$ to the node $v$, and exemplarily, the basic feature of the edge from the node $k$ to the node $v$ is obtained based on a distance between a sub-target object corresponding to the node $k$ and the sub-target object corresponding to the node $v$. When the type of the first target object is a protein, a distance between an amino acid corresponding to the node $k$ and the amino acid corresponding to the node $v$ refers to a standard spatial distance. In the formula 3, $e_{vk}$ is an associated feature ($\mu_{attached}$) in a message passing process. $h_v^{d+1}$ represents an updated node feature of the node $v$ through $d+1$ steps, and also represents a value of a node update function of a $(d+1)^{th}$ step corresponding to the node $v$, and the node update function uses linear transformation plus bias; $W_{in}$ and $W_a$ are parameters of the first node feature update layer, and in an exemplary embodiment, in the process of invoking the first node feature update layer to perform a plurality of steps of update, such two parameters as $W_{in}$ and $W_a$ are shared.

Assuming that the reference quantity is D (D is an integer not less than 1), after D steps of update are performed according to the formula 3, the target node feature including an updated node feature of each node in the graph information of the first target object is obtained, and step B is performed.

Step B: Obtain the node-level first basic feature information by invoking the first node-level feature output layer to output the target node feature, and.

The node-level first basic feature information includes a node-level output feature corresponding to each node in the graph information of the first target object.

The processing of the first node-level feature output layer may be considered as an additional message passing step, and in the first node-level feature output layer, the node-level first basic feature information corresponding to the first target object is obtained using different parameters. In an exemplary embodiment, the process of invoking the first node-level feature output layer to output the target node feature is implemented based on a formula 4:

$$m_v^o = \sum_{k \in N(v) \cup \{v\}} cat(h_k^D, x_k)$$
$$h_v^o = \sigma(W_0 m_v^o)$$

$$(\text{formula 4})$$

where $m_v^o$ represents a node-level fusion feature of the node $v$; $h_k^D$ represents an updated node feature of a node $k$ through $D$ steps; $x_k$ represents a basic feature of the node $k$; in the formula 4, the node $k$ is a node in a union set of the set $N(v)$ representing the adjacent nodes of the node $v$ and the node $v$; $h_v^o$ represents a node-level output feature corresponding to the node $v$; $W_o$ represents a parameter of the first node-level feature output layer; and meanings of other parameters are the same as those in the formula 3.

Based on the formula 4, the node-level output feature corresponding to each node in the graph information of the first target object can be obtained, and then the node-level first basic feature information corresponding to the first target object is obtained.

Exemplarily, the node-level first basic feature information corresponding to the first target object is represented as $H_a^1 = [h_1^o, h_2^o, \cdots, h_n^o]$, where n (n is an integer not less than 1) is the quantity of sub-target objects included in the first target object, and is also the quantity of nodes in the graph information of the first target object.

In a possible implementation, the first edge message passing model includes a first edge feature update layer and a first edge-level feature output layer, and the process of obtaining the edge-level first basic feature information by invoking the first edge message passing model to perform edge-level feature extraction on the graph information of the first target object, and includes the following step a and step b:

Step a: obtain a target edge feature, by invoking the first edge feature update layer to update an edge feature in the graph information of the first target object.

The target edge feature includes an updated edge feature of each edge in the graph information of the first target object obtained after processing through the first edge feature update layer. The first edge feature update layer in the first node message passing model is used for updating edge features in the graph information of the first target object by performing information aggregation on edges. In an exemplary embodiment, the process of invoking the first edge feature update layer to update an edge feature in the graph information of the first target object is implemented based on a formula 5:

$$h_{vw}^0 = \sigma\left(W'_{in} e_{vw}\right)$$

$$m_{vw}^{d+1} = \sum_{k \in N(v) \setminus w} cat\left(h_{vk}^d, x_k\right)$$

$$h_{vw}^{d+1} = \sigma\left(W_b m_{vw}^{d+1} + h_{vw}^0\right)$$

(formula 5)

where $h_{vw}^0$ represents an initial edge feature of an edge from the node $w$ to the node $v$; $e_{vw}$ represents a basic feature of an edge from the node $w$ to the node $v$; $m_{vw}^{d+1}$ represents a value of a message passing function of a $(d+1)^{th}$ ($d$ is an integer not less than 0) step corresponding to an edge from the node $w$ to the node $v$; $h_{vk}^d$ represents an updated edge feature of the edge from the node $k$ to the node $v$ through $d$ steps; $x_k$ represents a basic feature of the node $k$. $m_{vw}^{d+1}$ is calculated by aggregating features of an adjacent edge set of the edge from the node $w$ to the node $v$ and features of nodes corresponding to adjacent edges in the adjacent edge set. The adjacent edge set of the edge from the node $w$ to the node $v$ refers to a set of all edges starting from the node $v$ except the edge from the node $w$ to the node $v$. In the formula 5, $x_k$ is an associated feature ($\mu_{attached}$) in a message passing process. $h_{vw}^{d+1}$ represents an updated edge feature of the edge from the node $w$ to the node $v$ through $d+1$ steps, $W'_{in}$ and $W_b$ are parameters of the first edge feature update layer, and in an exemplary embodiment, in the process of invoking the first edge feature update layer to perform a plurality of steps of update, such two parameters as $W'_{in}$ and $W_b$ are shared.

In an exemplary embodiment, the update step quantity in the process of invoking the first edge feature update layer to update the edge features in the graph information of the first target object is the same as the update step quantity in the process of invoking the first node feature update layer to update the node features in the graph information of the first target object. Assuming that the same update step quantity is D (D is an integer not less than 1), after D steps of update are performed according to the formula 5, the target edge feature including an updated edge feature of each edge in the graph information of the first target object is obtained, and step b is performed.

Step b: obtain the edge-level first basic feature information, by invoking the first edge-level feature output layer to output the target edge feature, and.

The edge-level first basic feature information includes an edge-level output feature corresponding to each node in the graph information of the first target object.

The processing of the first edge-level feature output layer may be considered as a node information aggregation step. In the first edge-level feature output layer, the feature of the edge is transferred to the node, and then the edge-level first basic feature information is obtained. In an exemplary embodiment, the process of invoking the first edge-level feature output layer to output the target edge feature is implemented based on a formula 6:

$$m_v^{o'} = \sum_{k \in N(v)} cat\left(h_{kv}^D, x_k\right)$$

$$h_v^{o'} = \sigma\left(W'_0 m_v^{o'}\right)$$

(formula 6)

where $m_v^{o'}$ represents an edge-level fusion feature of the node $v$; $h_{kv}^D$ represents an updated edge feature of the edge from the node $k$ to the node $v$ through $D$ steps; $x_k$ represents a basic feature of the node $k$; $h_v^{o'}$ represents an

edge-level output feature corresponding to the node $v$; $W_0'$ represents a parameter of the first edge-level feature output layer.

Based on the formula 6, the edge-level output feature corresponding to each node in the graph information of the first target object can be obtained, and then the edge-level first basic feature information corresponding to the first target object is obtained.

Exemplarily, the edge-level first basic feature information corresponding to the first target object is represented as $H_b^1 = [h_1^{o'}, h_2^{o'}, \cdots, h_n^{o'}]$ , where n (n is an integer not less than 1) is the quantity of sub-target objects included in the first target object, and is also the quantity of nodes in the graph information corresponding to the first target object.

After the node-level first basic feature information and the edge-level first basic feature information corresponding to the first target object are obtained, the first basic feature information corresponding to the first target object is obtained.

The above description is only an exemplary description of obtaining the first basic feature information corresponding to the first target object based on the first feature extraction model, and this application is not limited thereto. In an exemplary embodiment, if the first feature extraction model is a single-branched message passing model, the first basic feature information corresponding to the first target object is basic feature information as a whole.

Step 2021b: Obtain, based on the first basic feature information corresponding to the first target object, first attention information corresponding to the first target object.

The first attention information includes attention information of each node in the graph information of the first target object, and attention information of any node includes one or more attention weights corresponding to the node at one or more angles. The quantity of the one or more angles of the one or more attention weights corresponding to the node and included in the attention information of the any node is not limited in this embodiment of this application, and the quantity may be set according to experience, or flexibly adjusted according to an application scenario. Exemplarily, the quantity of the one or more angles of the one or more attention weights corresponding to the node and included in the attention information of the any node is denoted as r (r is an integer not less than 1). In an exemplary embodiment, a sum of attention weights corresponding to the nodes in the graph information of the first target object at the same angle is 1.

The first attention information corresponding to the first target object is obtained based on the first basic feature information corresponding to the first target object. In a possible implementation, for the case that the first basic feature information includes the node-level first basic feature information and the edge-level first basic feature information, the first attention information corresponding to the first target object includes node-level first attention information and edge-level first attention information. In this case, the process of obtaining, based on the first basic feature information corresponding to the first target object, first attention information corresponding to the first target object is: obtaining, based on the node-level first basic feature information, the node-level first attention information corresponding to the first target object; and obtaining, based on the edge-level first basic feature information, the edge-level first attention information corresponding to the first target object.

The node-level first attention information includes node-level attention information of each node in the graph information of the first target object, and the edge-level first attention information includes edge-level attention information of each node in the graph information of the first target object. For any node in the graph information of the first target object, node-level attention information of the any node may be the same as edge-level attention information of the any node, or may be different from edge-level attention information of the any node. This is not limited in this embodiment of this application.

In a possible implementation, the process of obtaining, based on the node-level first basic feature information, the node-level first attention information corresponding to the first target object is implemented based on a formula 7:

$$S_a^1 = soft \max \left[ W_2 \tanh \left( W_1 H_a^{1T} \right) \right] \quad \text{(formula 7)}$$

where $S_a^1$ represents the node-level first attention information corresponding to the first target object; $H_a^{1T}$ represents a transpose of the node-level first basic feature information $H_a^1$ ; $W_1$ and $W_2$ are both learnable parameters; tanh(·) represents a hyperbolic tangent function. In an exemplary embodiment, $H_a^1 \in R^{n \times a}$ , $W_1 \in R^{h \times a}$, and $W_2 \in R^{r \times h}$.

Therefore, in the foregoing formula 7, $W_1$ is used for performing linear transformation, to transform the node-level first basic feature information in an a-dimensional space into that in an h-dimensional space, then nonlinear mapping is performed through the hyperbolic tangent function tanh(·), and then a feature in the h-dimensional space are linearly transformed into that in an r-dimensional (r is an integer not less than 1) space through $W_2$, to obtain a distribution of attention weights of a node at r different angles. For any angle, a larger attention weight corresponding to a node at the angle indicates that the node is more important at the angle. Finally, a sum of attention weights at all angles is caused to be 1 through the *soft*max(·) function.

In a possible implementation, the process of obtaining, based on the edge-level first basic feature information, the edge-level first attention information corresponding to the first target object is implemented based on a formula 8:

$$S_b^1 = soft\max\left[W_2\tanh\left(W_1 H_b^{1T}\right)\right] \quad \text{(formula 8)}$$

where $S_b^1$ represents the edge-level first attention information corresponding to the first target object; $H_b^{1T}$ represents a transpose of the edge-level first basic feature information $H_b^1$; meanings of other parameters are the same as those in the formula 7. In an exemplary embodiment, such two parameters as $W_1$ and $W_2$ in the formula 8 and such two parameters as $W_1$ and $W_2$ in the formula 7 are shared, so that information exchange can be made between the node-level first basic feature information and the edge-level first basic feature information during training.

After the node-level first attention information and the edge-level first attention information are obtained, the first attention information corresponding to the first target object is obtained. In an exemplary embodiment, the foregoing formula 7 and formula 8 may be considered as self-attention read functions, and through the self-attention read functions, the first attention information corresponding to the first target object can be obtained.

The above description is only an exemplary description of obtaining, based on the first basic feature information, the first attention information corresponding to the first target object, and this embodiment of this application is not limited thereto. When the first basic feature information is in other cases different from the foregoing cases, the process of obtaining the first attention information also changes.

In a possible implementation, the process of obtaining, based on the basic information of the second target object, second basic feature information and second attention information corresponding to the second target object includes the following step 2021c and step 2021d:

Step 2021c: Obtain, based on the basic information of the second target object, second basic feature information corresponding to the second target object.

In a possible implementation, the target interaction information prediction model further includes a second feature extraction model, and the second feature extraction model is used for extracting the second basic feature information corresponding to the second target object based on the basic information of the second target object. Based on this, the process of obtaining, based on the basic information of the second target object, second basic feature information corresponding to the second target object is: invoking the second feature extraction model to perform feature extraction on the basic information of the second target object, and obtaining the second basic feature information corresponding to the second target object. The model structure of the second feature extraction model is not limited in this embodiment of this application, as long as the model structure can determine the second basic feature information corresponding to the second target object based on the basic information of the second target object. In an exemplary embodiment, the model structure of the second feature extraction model is the same as the model structure of the first feature extraction model, and when the model structure of the first feature extraction model is a dual-message passing model formed by a node message passing model and an edge message passing model, the model structure of the second feature extraction model is similarly a dual-message passing model formed by a node message passing model and an edge message passing model.

In a possible implementation, the basic information of the second target object is the graph information of the second target object, the second feature extraction model includes a second node message passing model and a second edge message passing model, and the second basic feature information includes node-level second basic feature information and edge-level second basic feature information. In this case, the process of obtaining the second basic feature information corresponding to the second target object by invoking the second feature extraction model to perform feature extraction on the basic information of the second target object, includes: invoking the second node message passing model to perform node-level feature extraction on the graph information of the second target object, and obtaining the node-level second basic feature information; and invoking the second edge message passing model to perform edge-level feature extraction on the graph information of the second target object, and obtaining the edge-level second basic feature information. In an exemplary embodiment, the model structure of each of the second node message passing model and the second edge message passing model is an MPNN.

In a possible implementation, the second node message passing model includes a second node feature update layer and a second node-level feature output layer, and the process of obtaining the node-level second basic feature information, by invoking the second node message passing model to perform node-level feature extraction on the graph information of the second target object, includes: obtaining a target node feature; and invoking the second node-level feature output layer to output the target node feature, and obtaining the node-level second basic feature information, by invoking the second node feature update layer to update a node feature in the graph information of the second target object. For the implementation of the process, reference is made to step A and step B in step 2021a, and details are not described herein again.

In a possible implementation, the second edge message passing model includes a second edge feature update layer and a second edge-level feature output layer, and the process of obtaining the edge-level second basic feature information by invoking the second edge message passing model to perform edge-level feature extraction on the graph information of the second target object, is: obtaining a target edge feature by invoking the second edge feature update layer to update an edge feature in the graph information of the second target object; and obtaining the edge-level second basic feature information by invoking the second edge-level feature output layer to output the target edge feature. For the implementation of the process, reference is made to step a and step b in step 2021a, and details are not described herein again.

After the node-level second basic feature information and the edge-level second basic feature information corresponding to the second target object are obtained, the second basic feature information corresponding to the second target object is obtained.

The above description is only an exemplary description of obtaining the second basic feature information corresponding to the second target object based on the second feature extraction model, and this application is not limited thereto. In an exemplary embodiment, if the second feature extraction model is a single-branched message passing model, the second basic feature information corresponding to the second target object is basic feature information as a whole.

Step 2021d: Obtain, based on the second basic feature information corresponding to the second target object, second attention information corresponding to the second target object.

The second attention information includes attention information of each node in the graph information of the second target object, and the second attention information corresponding to the second target object is obtained based on the second basic feature information corresponding to the second target object.

In a possible implementation, for the case that the second basic feature information includes the node-level second basic feature information and the edge-level second basic feature information, the second attention information corresponding to the second target object includes node-level second attention information and edge-level second attention information. In this case, the process of obtaining, based on the second basic feature information, second attention information corresponding to the second target object is: obtaining, based on the node-level second basic feature information, the node-level second attention information corresponding to the second target object; and obtaining, based on the edge-level second basic feature information, the edge-level second attention information corresponding to the second target object. For the implementation of the process, reference is made to step 2021b, and details are not described herein again. After the node-level second attention information and the edge-level second attention information are obtained, the second attention information corresponding to the second target object is obtained.

Parameter values in the self-attention read function used in the process of obtaining the node-level second attention information and the edge-level second attention information corresponding to the second target object may be different from or the same as parameter values in the self-attention read function used in the process of obtaining the node-level first attention information and the edge-level first attention information corresponding to the first target object. This is not limited in this embodiment of this application.

The node-level second attention information includes node-level attention information of each node in the graph information of the second target object, and the edge-level second attention information includes edge-level attention information of each node in the graph information of the second target object. For any node in the graph information of the second target object, node-level attention information of the any node may be the same as edge-level attention information of the any node, or may be different from edge-level attention information of the any node. This is not limited in this embodiment of this application.

The above description is only an exemplary description of obtaining, based on the second basic feature information, the second attention information corresponding to the second target object, and this embodiment of this application is not limited thereto. When the second basic feature information is in other cases different from the foregoing cases, the process of obtaining the second attention information also changes.

Step 2022: Obtain, based on the first basic feature information and the first attention information, first global feature information corresponding to the first target object; and obtain, based on the second basic feature information

and the second attention information, second global feature information corresponding to the second target object.

The first global feature information corresponding to the first target object refers to global feature information including attention information and corresponding to the first target object. In a possible implementation, the first basic feature information includes node-level first basic feature information and edge-level first basic feature information, and the first attention information includes node-level first attention information and edge-level first attention information. In this case, the first global feature information corresponding to the first target object includes node-level first global feature information and edge-level first global feature information.

In a possible implementation, the process of obtaining, based on the first basic feature information and the first attention information, first global feature information corresponding to the first target object is: obtaining, based on the node-level first basic feature information and the node-level first attention information, node-level first global feature information corresponding to the first target object; and obtaining, based on the edge-level first basic feature information and the edge-level first attention information, edge-level first global feature information corresponding to the first target object.

The node-level first global feature information corresponding to the first target object refers to global feature information including node-level attention information and corresponding to the first target object. In a possible implementation, the process of obtaining, based on the node-level first basic feature information and the node-level first attention information, node-level first global feature information corresponding to the first target object is implemented based on a formula 9:

$$\xi_a^1 = flatten\left(S_a^1 H_a^1\right) \quad \text{(formula 9)}$$

where $\xi_a^1$ represents the node-level first global feature information corresponding to the first target object; $H_a^1$ represents the node-level first basic feature information corresponding to the first target object; $S_a^1$ represents the node-level first attention information corresponding to the first target object; *flatten*($\cdot$) represents flattening into a one-dimensional vector. In an exemplary embodiment, $H_a^1 \in R^{n \times a}$, and $S_a^1 \in R^{r \times n}$, and therefore $S_a^1 H_a^1 \in R^{r \times a}$, where n represents the quantity of nodes in the graph information of the first target object, r represents the quantity of angles of attention weights corresponding to any node, and a represents a customized alignment value. Even if the quantities of nodes in different graph information of the first target objects are different, global feature information including attention weights of nodes and fixed in size can still be obtained based on the formula 9.

The edge-level first global feature information corresponding to the first target object refers to global feature information including edge-level attention information and corresponding to the first target object. In a possible implementation, the process of obtaining, based on the edge-level first basic feature information and the edge-level first attention information, edge-level first global feature information corresponding to the first target object is implemented based on a formula 10:

$$\xi_b^1 = flatten\left(S_b^1 H_b^1\right) \quad \text{(formula 10)}$$

where $\xi_b^1$ represents the edge-level first global feature information corresponding to the first target object; $H_b^1$ represents the edge-level first basic feature information corresponding to the first target object; $S_b^1$ represents the edge-level first attention information corresponding to the first target object.

After the node-level first global feature information and the edge-level first global feature information are obtained, the first global feature information corresponding to the first target object is obtained.

The second global feature information corresponding to the second target object refers to global feature information including attention information and corresponding to the second target object. In a possible implementation, the second basic feature information includes node-level second basic feature information and edge-level second basic feature information, and the second attention information includes node-level second attention information and edge-level second attention information. In this case, the second global feature information corresponding to the second target object includes node-level second global feature information and edge-level second global feature information.

In a possible implementation, the process of obtaining, based on the second basic feature information and the second attention information, second global feature information corresponding to the second target object is: obtaining, based on the node-level second basic feature information and the node-level second attention information, node-level second global feature information corresponding to the second target object; and obtaining, based on the edge-level second basic feature information and the edge-level second attention information, edge-level second global feature information corresponding to the second target object.

The node-level second global feature information corresponding to the second target object refers to global feature information including node-level attention information and corresponding to the second target object. The edge-level second global feature information corresponding to the second target object refers to global feature information including edge-level attention information and corresponding to the second target object. For the manner of obtaining the node-level second global feature information and the edge-level second global feature information corresponding to the second target object, reference is made to the manner of obtaining the node-level first global feature information and the edge-level first global feature information corresponding to the first target object, and details are not described herein again. After the node-level second global feature information and the edge-level second global feature information are obtained, the second global feature information corresponding to the second target object is obtained.

The above description is only an exemplary description of obtaining the first global feature information corresponding to the first target object and the second global feature information corresponding to the second target object, and this embodiment of this application is not limited thereto. In an exemplary embodiment, for the case that each of the first basic feature information and the second basic feature information is basic feature information as a whole, each of the first attention information and second attention information is similarly attention information as a whole. In this case, the first global feature information as a whole is obtained directly based on the first basic feature information and the first attention information, and the second global feature information as a whole is obtained directly based on the second basic feature information and second attention information.

Because features of all nodes in the graph information of the first target object are comprehensively considered in the process of obtaining the first global feature information, the first global feature information can globally represent the first target object, and similarly, the second global feature information can globally represent the second target object.

Step 2023: Obtain, based on the first global feature information and the second global feature information, the target interaction information between the first target object and the second target object.

In a possible implementation, the target interaction information prediction model includes a prediction processing model, and the implementation process of obtaining, based on the first global feature information and the second global feature information, the target interaction information between the first target object and the second target object is: obtaining target interaction information between the first target object and the second target object, by invoking the prediction processing model to predict the first global feature information and the second global feature information.

After the first global feature information corresponding to the first target object and the second global feature information corresponding to the second target object are obtained, the first global feature information and the second global feature information are inputted to the prediction processing model, and processed through the prediction processing model, to obtain the target interaction information between the first target object and the second target object. In an exemplary embodiment, after the first global feature information and the second global feature information are inputted to the prediction processing model, the prediction processing model integrates the first global feature information and the second global feature information, and then obtains the target interaction information between the first target object and the second target object.

In a possible implementation, for the case that the first global feature information includes the node-level first global feature information and the edge-level first global feature information, and the second global feature information includes the node-level second global feature information and the edge-level second global feature information, the target interaction information between the first target object and the second target object includes node-level target interaction information and edge-level target interaction information, and the prediction processing model includes a first prediction processing model and a second prediction processing model. The first prediction processing model is used for obtaining the node-level target interaction information according to the node-level global feature information, and the second prediction processing model is used for obtaining the edge-level target interaction information according to the edge-level global feature information.

In the foregoing case, the process of obtaining target interaction information between the first target object and the second target object by invoking the prediction processing model to predict the first global feature information and the second global feature information, is: obtaining node-level target interaction information between the first target object and the second target object by invoking the first prediction processing model to predict the node-level first global feature information and the node-level second global feature information; and obtaining edge-level target interaction information between the first target object and the second target object by invoking the second prediction processing model to predict the edge-level first global feature information and the edge-level second global feature information.

In a possible implementation, the first prediction processing model refers to a fully connected neural network, and the process of obtaining node-level target interaction information between the first target object and the second target object by invoking the first prediction processing model to predict the node-level first global feature information and the node-level second global feature information, is implemented based on a formula 11:

$$Pred_a = FCN_a[cat(\xi_a^1, \xi_a^2)]$$ (formula 11)

where $Pred_a$ represents the node-level target interaction information between the first target object and the second target object; $\xi_a^1$ represents the node-level first global feature information corresponding to the first target object and used for representing the node-level global feature corresponding to the first target object and obtained after processing through the first node message passing model and the self-attention read function; $\xi_a^2$ represents the node-level second global feature information corresponding to the second target object and used for representing the node-level global feature corresponding to the second target object and obtained after processing through the second node message passing model and the self-attention read function; $cat(\cdot,\cdot)$ represents a concatenating function, which concatenates the node-level first global feature information corresponding to the first target object and the node-level second global feature information corresponding to the second target object, to combine the node-level information of the first target object and the node-level information of the second target object; $FCN_a$ represents a parameter of the first prediction processing model.

In a possible implementation, the second prediction processing model refers to a fully connected neural network, and the process of obtaining edge-level target interaction information between the first target object and the second target object by invoking the second prediction processing model to predict the edge-level first global feature information and the edge-level second global feature information, is implemented based on a formula 12:

$$Pred_b = FCN_b[cat(\xi_b^1, \xi_b^2)]$$ (formula 12)

where $Pred_b$ represents the edge-level target interaction information between the first target object and the second target object; $\xi_b^1$ represents the edge-level first global feature information corresponding to the first target object and used for representing the edge-level global feature corresponding to the first target object and obtained after processing through the first edge message passing model and the self-attention read function; $\xi_b^2$ represents the edge-level second global feature information corresponding to the second target object and used for representing the edge-level global feature corresponding to the second target object and obtained after processing through the second edge message passing model and the self-attention read function; $cat(\cdot,\cdot)$ represents a concatenating function, which concatenates the edge-level first global feature information corresponding to the first target object and the edge-level second global feature information corresponding to the second target object, to combine the node-level information of the first target object and the edge-level information of the second target object; $FCN_b$ represents a parameter of the second prediction processing model.

In an exemplary embodiment, the model structure of the second prediction processing model and the model structure of the first prediction processing model are the same as each other and are both fully connected neural networks, but because the second prediction processing model and the first prediction processing model process different information, the parameter of the second prediction processing model may be different from the parameter of the first prediction processing model.

After the node-level target interaction information and the edge-level target interaction information are obtained, the target interaction information between the first target object and the second target object is obtained.

The above description is only an exemplary description of obtaining the target interaction information between the first target object and the second target object, and this embodiment of this application is not limited thereto. In an exemplary embodiment, for the case that each of the first global feature information and the second global feature information is global feature information as a whole, the prediction processing model is also a processing model as a whole. In this case, the prediction processing model is directly invoked to predict the first global feature information and the second global feature information, to obtain the target interaction information as a whole.

In a possible implementation, after the obtaining the first attention information corresponding to the first target object and the second attention information corresponding to the second target object, the method further includes: determining, based on the first attention information corresponding to the first target object, a first key sub-target object in all sub-target objects of the first target object, the first key sub-target object being used for indicating a sub-target object that is in the first target object and that is used for interacting with the second target object; and determining, based on the second attention information corresponding to the second target object, a second key sub-target object in all sub-target objects of the second target object, the second key sub-target object being used for indicating a sub-target object that is in the second target object and that is used for interacting with the first target object.

In a possible implementation, the manner of determining, based on the first attention information corresponding to the first target object, a first key sub-target object in all sub-target objects of the first target object is: determining, based on the first attention information corresponding to the first target object, attention information corresponding to each sub-target object in the first target object; and using a sub-target object that is of all sub-target objects included in the first target object and whose corresponding attention information meets a selection condition as a first key sub-target object. The first key sub-target object is used for indicating a sub-target object that is in the first target object and that is used for interacting with the second target object.

In a possible implementation, using an example in which the first attention information corresponding to the first target object is attention information as a whole, attention information corresponding to any sub-target object in the first target object is similarly attention information as a whole. Attention information corresponding to any sub-target object in the first target object includes attention weights of the any sub-target object at angles. Based on this, that attention information corresponding to any sub-target object in the first target object meets the selection condition means that attention weights not less than a target proportion of the attention weights of the any sub-target object at the angles meet a threshold condition, and meeting the threshold condition refers to being not less than a weight threshold. The target proportion and the weight threshold are set according to experience, or flexibly adjusted according to an application scenario. This is not limited in this embodiment of this application.

Exemplarily, the target proportion is set to 80%, and the weight threshold is set to 0.3. Assuming that attention information corresponding to any sub-target object in the first target object includes attention weights of the any sub-target object at 10 angles, when eight or more attention weights of the attention weights of the any sub-target object at the 10 angles are not less than 0.3, it indicates that the attention information corresponding to the any sub-target object meets the selection condition.

In a possible implementation, for the case that the first attention information corresponding to the first target object includes the node-level first attention information and the edge-level first attention information, attention information corresponding to any sub-target object in the first target object similarly includes node-level attention information and edge-level attention information. Each of the node-level attention information and the edge-level attention information corresponding to any sub-target object in the first target object includes attention weights of the any sub-target object at angles. Based on this, that attention information corresponding to any sub-target object in the first target object meets the selection condition may mean that the node-level attention information corresponding to the any sub-target object meets the selection condition, or may mean that the edge-level attention information corresponding to the any sub-target object meets the selection condition, or may mean that the node-level attention information and the edge-level attention information corresponding to the any sub-target object both meet the selection condition. This is not limited in this embodiment of this application.

For the implementation of determining, based on the second attention information corresponding to the second target object, a second key sub-target object in all sub-target objects of the second target object, reference is made to the foregoing implementation of determining, based on the first attention information corresponding to the first target object, a first key sub-target object in all sub-target objects of the first target object, and details are not described herein again.

During actual prediction, sub-target objects in a target object which interact with another target object are unknown. Key sub-target objects determined in all sub-target objects included in a target object are used for indicating sub-target objects in the target object which interact with another target object.

Because the key local-level loss function determined based on attention information corresponding to one or more key sub-sample objects in one or more sample objects meeting a reference condition is used in the process of training the interaction information prediction model, reliability of the first attention information of the first target object and the second attention information of the second target object obtained by using the target interaction information prediction model is relatively high, and key sub-target objects determined based on attention information corresponding to a target object can relatively accurately indicate sub-target objects in the target object which interact with another target object.

In an exemplary embodiment, attention information corresponding to a target object may indicate that no key sub-target object exists in all sub-target objects of the target object. In this case, it indicates that the target object does not meet the reference condition. In this case, it is considered that the target object as a whole interacts with another target object.

In an exemplary embodiment, the type of one target object of the first target object and the second target object is a protein, and the type of the other target object is a small molecule. The using the target interaction information prediction model to predict the target interaction information between the first target object and the second target object can be applied to a scenario of screening a small molecule. In this case, the target interaction information prediction model may be considered as a drug screening model, a drug refers to a small molecule interacting with a protein, and interaction information may be considered as protein-small molecule active information.

Exemplarily, a process of obtaining the target interaction information between the first target object and the second target object is shown in FIG. 5. Graph information (only partial graph information is shown in FIG. 5) of a first target object 501 is inputted to a first feature extraction model 502 in a target interaction information prediction model, subjected to feature extraction, and processed by a self-attention read function, to obtain first global feature information 503 corresponding to the first target object; graph information (only partial graph information is shown in FIG. 5) of a second target object 504 is inputted to a second feature extraction model 505 in the target interaction information prediction model, subjected to feature extraction, and processed by a self-attention read function, to obtain second global feature information 506 corresponding to the second target object; and a prediction processing model in the target interaction information prediction model is invoked to integrate the first global feature information 503 corresponding to the first target object and the second global feature information 506 corresponding to the second target object, to obtain target interaction information between the first target object and the second target object based on integrated feature information 507.

In the embodiments of this application, the global-level loss function and the key local-level loss function are used for training the interaction information prediction model, and then the trained interaction information prediction model is used for determining the interaction information between the first target object and the second target object. The global-level loss function can cause the model training process to pay attention to the global information; and the key local-level loss function can cause the model training process to pay attention to the key local information. That is to say, in the embodiments of this application, the process of training the interaction information prediction model not only pays attention to the global information, but also pays attention to the key local information, so that the effect of training the interaction information prediction model is relatively good, and the accuracy of the interaction information between the first target object and the second target object determined by using the trained interaction information prediction model is relatively high.

Based on the foregoing implementation environment shown in FIG. 1, an embodiment of this application provides an interaction information prediction model training method, the interaction information prediction model training method is performed by a computer device, and the computer device may be the terminal 11, or may be the server 12. In this embodiment of this application, a description is made using an example in which the interaction information prediction model training method is applied to the server 12. As shown in FIG. 6, the interaction information prediction model training method provided in this embodiment of this application may include the following step 601 to step 606:

Step 601. Invoke an initial interaction information prediction model, and obtain attention information corresponding to a first sample object, attention information corresponding to a second sample object, and predictive interaction information between the first sample object and the second sample object.

The predictive interaction information is obtained based on global feature information corresponding to the first sample object and global feature information corresponding to the second sample object.

The initial interaction information prediction model refers to a model that needs to be trained and that is used for predicting interaction information, and the manner of obtaining the initial interaction information prediction model is not limited in this embodiment of this application. Exemplarily, the initial interaction information prediction model is designed by developers and uploaded to a server, so that the server obtains the initial interaction information prediction model. A parameter of the initial interaction information prediction model is a to-be-updated parameter, and the initial interaction information prediction model is trained by updating the parameter of the initial interaction information prediction model.

The first sample object and the second sample object are a group of sample objects used for training the initial interaction information prediction model. In an exemplary embodiment, in the process of training the initial interaction information prediction model, a plurality of groups of sample objects may be used. In this embodiment of this application, a description is made using an example in which a group of sample objects is used for training the initial interaction information prediction model. In a possible implementation, the type of the first sample object is the same as the type of the first target object in the embodiment shown in FIG. 2, and the type of the second sample object is the same as the type of the second target object in the embodiment shown in FIG. 2, to ensure accuracy of interaction information predicted by a target interaction information prediction model.

In a possible implementation, the process of obtaining attention information corresponding to a first sample object, attention information corresponding to a second sample object, and predictive interaction information between the first sample object and the second sample object, by invoking an initial interaction information prediction model, is: obtaining, based on basic information of the first sample object, basic feature information corresponding to the first sample object and the attention information corresponding to the first sample object by invoking the initial interaction information prediction model; obtaining, based on basic information of the second sample object, basic feature information corresponding to the second sample object and the attention information corresponding to the second sample object; obtaining, based on the basic feature information corresponding to the first sample object and the attention information corresponding to the first sample object, the global feature information corresponding to the first sample object; obtaining, based on the basic feature information corresponding to the second sample object and the attention information corresponding to the second sample object, the global feature information corresponding to the second sample object; and obtaining, based on the global feature information corresponding to the first sample object and the global feature information corresponding to the second sample object, the predictive interaction information between the first sample object and the second sample object. For the implementation of the process, reference is made to step 2021 to step 2023 in the embodiment shown in FIG. 2, and details are not described herein again.

Step 602. Obtain, based on the predictive interaction information and standard interaction information between the first sample object and the second sample object, a global-level loss function.

The global-level loss function refers to a loss function obtained by paying attention to global information. The standard interaction information between the first sample object and the second sample object is used for indicating true interaction information between the first sample object and the second sample object. Exemplarily, the standard interaction information between the first sample object and the second sample object exists in the form of a label.

In a possible implementation, for the case that the predictive interaction information includes node-level predictive interaction information and edge-level predictive interaction information, the process of obtaining, based on the predictive interaction information and standard interaction information between the first sample object and the second sample object, a global-level loss function includes the following step 6021 to step 6024:

Step 6021: Determine, based on the node-level predictive interaction information and the standard interaction information between the first sample object and the second sample object, a first loss sub-function.

The first loss sub-function is used for measuring a difference between the node-level predictive interaction information and the standard interaction information, the form of the first loss sub-function is not limited in this embodiment of this application, and exemplarily, the form of the first loss sub-function is a mean square error.

In an exemplary embodiment, the process of determining, based on the node-level predictive interaction information and the standard interaction information between the first sample object and the second sample object, a first loss sub-function is implemented based on a formula 13:

$$L_{Pred_a} = MSE(Pred_a, Target) \quad \text{(formula 13)}$$

where $L_{Pred_a}$ represents the first loss sub-function; $Pred_a$ represents the node-level predictive interaction information; $Target$ represents the standard interaction information; $MSE(\cdot, \cdot)$ represents a mean square error function.

Step 6022: Determine, based on the edge-level predictive interaction information and the standard interaction information between the first sample object and the second sample object, a second loss sub-function.

The second loss sub-function is used for measuring a difference between the edge-level predictive interaction information and the standard interaction information, the form of the second loss sub-function is not limited in this embodiment of this application, and exemplarily, the form of the second loss sub-function is a mean square error.

In an exemplary embodiment, the process of determining, based on the edge-level predictive interaction information and the standard interaction information between the first sample object and the second sample object, a second loss sub-function is implemented based on a formula 14:

$$L_{Pred_b} = MSE(Pred_b, Target) \quad \text{(formula 14)}$$

where $L_{Pred_b}$ represents the second loss sub-function; $Pred_b$ represents the edge-level predictive interaction information; $Target$ represents the standard interaction information; $MSE(\cdot, \cdot)$ represents a mean square error function.

Step 6023: Determine, based on the node-level predictive interaction information and the edge-level predictive interaction information, a third loss sub-function.

The third loss sub-function is used for measuring a difference between the node-level predictive interaction information and the edge-level predictive interaction information, the form of the third loss sub-function is not limited in this embodiment of this application, and exemplarily, the form of the third loss sub-function is a mean square error.

In an exemplary embodiment, the process of determining, based on the node-level predictive interaction information and the edge-level predictive interaction information, a third loss sub-function is implemented based on a formula 15:

$$L_{dis} = MSE(Pred_a, Pred_b) \quad \text{(formula 15)}$$

where $L_{dis}$ represents the third loss sub-function; $Pred_a$ represents the node-level predictive interaction information; $Pred_b$ represents the edge-level predictive interaction information; $MSE(\cdot,\cdot)$ represents a mean square error function. The objective of designing the third loss sub-function is: reducing the difference between the node-level predictive interaction information and the edge-level predictive interaction information.

Step 6024: Determine, based on the first loss sub-function, the second loss sub-function, and the third loss sub-function, the global-level loss function.

After the first loss sub-function, the second loss sub-function, and the third loss sub-function are obtained, the global-level loss function is determined based on the first loss sub-function, the second loss sub-function, and the third loss sub-function, to obtain the loss function used for paying attention to the global information.

In an exemplary embodiment, the manner of determining, based on the first loss sub-function, the second loss sub-function, and the third loss sub-function, the global-level loss function is: performing weighted summation on the first loss sub-function, the second loss sub-function, and the third loss sub-function, to obtain the global-level loss function. During the weighted summation, a weight corresponding to each loss sub-function is set according to experience or flexibly adjusted according to an application scenario. This is not limited in this embodiment of this application. Exemplarily, a weight corresponding to each loss sub-function may be set to 1, and therefore the global-level loss function is a sum of the first loss sub-function, the second loss sub-function, and the third loss sub-function.

It can be learned according to the foregoing content that, the global-level loss function is obtained after integrating a plurality of loss sub-functions, and can perform multi-supervision on the process of training the interaction information prediction model.

The foregoing description of step 6021 to step 6024 is only an exemplary description of obtaining the global-level loss function, and this embodiment of this application is not limited thereto. In an exemplary embodiment, for the case that the predictive interaction information is predictive interaction information as a whole, the loss function determined based on the predictive interaction information and the standard interaction information is directly used as the global-level loss function.

Step 603. Determine, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object.

The one or more key sub-sample objects in the one or more sample objects meeting the reference condition are a part of all sub-sample objects of the one or more sample objects meeting the reference condition.

The one or more sample objects meeting the reference condition are at least one of the first sample object and the second sample object. That is to say, at least one of the first sample object and the second sample object meets the reference condition, that is, the first sample object meets the reference condition, or the second sample object meets the reference condition, or both the first sample object and the second sample object meet the reference condition. The one or more key sub-sample objects in the one or more sample objects meeting the reference condition are a part of all sub-sample objects of the one or more sample objects meeting the reference condition. In an exemplary embodiment, a key sub-sample object in a sample object refers to a sub-sample object that is of all sub-sample objects included in the sample object and that interacts with another sample object.

For one sample object of two sample objects, all sub-sample objects included in the sample object may interact with the other sample object, or only some sub-sample objects included in the sample object may interact with the other sample object. In this embodiment of this application, a sample object whose only some sub-sample objects of all the included sub-sample objects interact with another sample object is used as a sample object meeting the reference condition. Exemplarily, assuming that the type of one of two sample objects is a protein and the type of the other sample object is a small molecule, all sub-sample objects included in the sample object of the protein type are an amino acid 1, an amino acid 2, and an amino acid 3. If only one or two of the amino acid 1, the amino acid 2, and the amino acid 3 and interact with the sample object of the small molecule type, the sample object of the protein type is used as a sample object meeting the reference condition. If all of the amino acid 1, the amino acid 2, and the amino acid 3 and interact with the sample object of the small molecule type, the sample object of the protein type is used as a sample object not meeting the reference condition.

In the embodiments of this application, the two sample objects include at least one sample object meeting the reference condition. In an exemplary embodiment, when the first sample object and the second sample object are obtained, information used for indicating key sub-sample objects which the sample object meeting the reference condition includes can be further obtained, thereby directly determining the key sub-sample objects in the sample object meeting the reference condition. The information used for indicating the key sub-sample objects which the sample object meeting the reference condition includes may be considered as supervision information corresponding to the sample object meeting the reference condition, and the supervision information provides a reference for obtaining the key local-level loss function.

After the first sample object and the second sample object are obtained, one or more sample objects which meet the reference condition may be learned. After the sample object meeting the reference condition is determined, in attention information corresponding to the sample object meeting the reference condition, attention information corresponding to one or more key sub-sample objects in the sample object meeting the reference condition is determined.

Attention information corresponding to a sample object includes attention information of each node in graph information of the sample object, and attention information of any node includes one or more attention weights corresponding to the any node at one or more angles. Because each node in graph information of a sample object corresponds to a sub-sample object in the sample object, attention information of any node can be directly used as attention information corresponding to a sub-sample object corresponding to the any node. That is to say, after a key sub-sample object in the sample object meeting the reference condition is learned, attention information corresponding to the key sub-sample object can be directly determined in attention information corresponding to the sample object meeting the reference condition. There may be one or more key sub-sample objects in a sample object meeting the reference condition, and determining attention information corresponding to the one or more key sub-sample objects refers to determining attention information corresponding to each key sub-sample object respectively. Attention information corresponding to any key sub-sample object includes attention weights corresponding to the key sub-sample object at one or more angles.

Step 604. Obtain, based on the attention information corresponding to the one or more key sub-sample objects in the one or more sample objects meeting the reference condition, a key local-level loss function.

After the attention information corresponding to the one or more key sub-sample objects in the one or more sample objects meeting the reference condition is determined, the key local-level loss function is obtained based on the attention information corresponding to the one or more key sub-sample objects in the one or more sample objects meeting the reference condition. Because the one or more key sub-sample objects are a part of all sub-sample objects included in the one or more sample objects meeting the reference condition, the key local-level loss function obtained based on the attention information corresponding to the one or more key sub-sample objects can be used for causing the process of training the interaction information prediction model to pay attention to key local information.

In a possible implementation, the one or more sample objects meeting the reference condition include the following three cases. In different cases, implementations of step 604 are different.

Case 1: The one or more sample objects meeting the reference condition are the first sample object.

In this case 1, the implementation of step 603 is: determining, in the attention information corresponding to the first sample object, attention information corresponding to one or more key sub-sample objects in the first sample object. The implementation of step 604 is: determining, based on the attention information corresponding to the one or more key sub-sample objects in the first sample object, a key local-level loss function.

The attention information corresponding to the first sample object includes attention information of each node in graph information of the first sample object. Exemplarily, the manner of determining, in the attention information corresponding to the first sample object, attention information corresponding to one or more key sub-sample objects in the first sample object is: determining, in the attention information corresponding to the first sample object, attention information of one or more nodes corresponding to the one or more key sub-sample objects in the first sample object, and using the attention information of the one or more nodes corresponding to the one or more key sub-sample objects in the first sample object as the attention information corresponding to the one or more key sub-sample objects in the first sample object.

There are one or more key sub-sample objects in the first sample object. Attention information corresponding to any key sub-sample object includes attention weights corresponding to the key sub-sample object at one or more angles. Attention weights included in attention information corresponding to different key sub-sample objects in the first sample object correspond to the same angle. In an exemplary embodiment, because a sum of attention weights corresponding to nodes in the graph information of the first sample object at the same angle is 1, a sum of attention weights corresponding to sub-sample objects in the first sample object at the same angle is also 1.

In an exemplary embodiment, the process of determining, based on the attention information corresponding to the one or more key sub-sample objects in the first sample object, a key local-level loss function is implemented based on a formula 16:

$$L_{pocket} = \frac{1}{r} \sum\nolimits_{i=1}^{r} MSE\left[sum\left(W_{pocket}^{i}\right), 1\right]$$

(formula 16)

where $L_{pocket}$ represents the key local-level loss function determined in this case 1; $r$ represents the quantity of one or more angles of one or more attention weights corresponding to any key sub-sample object in the first sample object included in attention information corresponding to the key sub-sample object, where $r$ is an integer not less than 1;

$sum\left(W_{pocket}^{i}\right)$ represents a sum of attention weights corresponding to all key sub-sample objects in the first sample object at an $i^{th}$ ($i$ is a positive integer not greater than r) angle.

Case 2: The one or more sample objects meeting the reference condition are the second sample object.

In this case 2, the implementation of step 603 is: determining, in the attention information corresponding to the second sample object, attention information corresponding to one or more key sub-sample objects in the second sample object. The implementation of step 604 is: determining, based on the attention information corresponding to the one or more key sub-sample objects in the second sample object, a key local-level loss function.

The attention information corresponding to the second sample object includes attention information of each node in graph information of the second sample object. Exemplarily, the manner of determining, in the attention information corresponding to the second sample object, attention information corresponding to one or more key sub-sample objects in the second sample object is: determining, in the attention information corresponding to the second sample object, attention information of one or more nodes corresponding to the one or more key sub-sample objects in the second sample object, and using the attention information of the one or more nodes corresponding to the one or more key sub-sample objects in the second sample object as the attention information corresponding to the one or more key sub-sample objects in the second sample object.

There are one or more key sub-sample objects in the second sample object. Attention information corresponding to any key sub-sample object includes attention weights corresponding to the key sub-sample object at one or more angles. Attention weights included in attention information corresponding to different key sub-sample objects in the second sample object correspond to the same angle. In an exemplary embodiment, because a sum of attention weights corresponding to nodes in the graph information of the second sample object at the same angle is 1, a sum of attention weights corresponding to sub-sample objects in the second sample object at the same angle is also 1.

In an exemplary embodiment, the process of determining, based on the attention information corresponding to the one or more key sub-sample objects in the second sample object, a key local-level loss function is also implemented based on the foregoing formula 16, and when the process of determining, based on the attention information corresponding to the one or more key sub-sample objects in the second sample object, a key local-level loss function is implemented based on the formula 16, $L_{pocket}$ represents the key local-level loss function determined in this case 2; $r$ represents the quantity of one or more angles of one or more attention weights corresponding to any key sub-sample object in the second sample object included in attention information corresponding to the key sub-sample object, where

$r$ is an integer not less than 1; $sum\left(W_{pocket}^{i}\right)$ represents a sum of attention weights corresponding to all key sub-sample objects in the second sample object at an $i^{th}$ angle.

Case 3: The one or more sample objects meeting the reference condition are the first sample object and the second sample object.

In this case 3, the implementation of step 603 is: determining, in the attention information corresponding to the first sample object, attention information corresponding to a first key sub-sample object in the first sample object; and determining, in the attention information corresponding to the second sample object, attention information corresponding to a second key sub-sample object in the second sample object. The implementation of step 604 is: determining, based on the attention information corresponding to the first key sub-sample object, a fourth loss sub-function; determining, based on the attention information corresponding to the second key sub-sample object, a fifth loss sub-function; and determining, based on the fourth loss sub-function and the fifth loss sub-function, the key local-level loss function.

Exemplarily, the process of determining, based on the attention information corresponding to the first key sub-sample object, a fourth loss sub-function is implemented based on the formula 16, and when the process of determining, based on the attention information corresponding to the first key sub-sample object, a fourth loss sub-function is implemented based on the formula 16, $L_{pocket}$ represents the fourth loss sub-function; $r$ represents the quantity of one or more angles of one or more attention weights corresponding to any first key sub-sample object included in attention information corresponding to the any first key sub-sample object, where $r$ is an integer not less than 1; $sum\left(W_{pocket}^{i}\right)$ represents a sum of attention weights corresponding to all first key sub-sample objects at an $i^{th}$ angle.

Exemplarily, the process of determining, based on the attention information corresponding to the second key sub-sample object, a fifth loss sub-function is implemented based on the formula 16, and when the process of determining, based on the attention information corresponding to the second key sub-sample object, a fifth loss sub-function is implemented based on the formula 16, $L_{pocket}$ represents the fifth loss sub-function; $r$ represents the quantity of one or more angles of one or more attention weights corresponding to any second key sub-sample object included in attention information corresponding to the any second key sub-sample object, where $r$ is an integer not less than 1;

$sum\left(W^i_{pocket}\right)$ represents a sum of attention weights corresponding to all second key sub-sample objects at an $i^{th}$ angle.

After the fourth loss sub-function and the fifth loss sub-function are determined, the key local-level loss function is determined based on the fourth loss sub-function and the fifth loss sub-function. In an exemplary embodiment, the manner of determining, based on the fourth loss sub-function and the fifth loss sub-function, the key local-level loss function is: performing weighted summation on the fourth loss sub-function and the fifth loss sub-function, to obtain the key local-level loss function. During the weighted summation, weights corresponding to the fourth loss sub-function and the fifth loss sub-function are set according to experience or flexibly adjusted according to an application scenario. This is not limited in this embodiment of this application. Exemplarily, a weight corresponding to each loss sub-function may be set to 1, and therefore the key local-level loss function is a sum of the fourth loss sub-function and the fifth loss sub-function.

The implementation of step 604 is described in the foregoing content using an example in which attention information corresponding to a sample object is attention information as a whole. This embodiment of this application is not limited thereto. In an exemplary embodiment, for the case that attention information corresponding to a sample object includes node-level attention information and edge-level attention information, attention information corresponding to a key sub-sample object includes node-level attention information and edge-level attention information. In this case, the process of obtaining, based on the attention information corresponding to the key sub-sample object, the key local-level loss function may be: obtaining, based on the node-level attention information corresponding to the key sub-sample object, the key local-level loss function; or may be: obtaining, based on the edge-level attention information corresponding to the key sub-sample object, the key local-level loss function; or may be: obtaining, based on the node-level attention information and the edge-level attention information corresponding to the key sub-sample object, the key local-level loss function. This is not limited in this embodiment of this application.

In an exemplary embodiment, for both the process of obtaining, based on the node-level attention information corresponding to the key sub-sample object, the key local-level loss function and the process of obtaining, based on the edge-level attention information corresponding to the key sub-sample object, the key local-level loss function, reference may be made to the process of obtaining the key local-level loss function in the case that the attention information is attention information as a whole.

In an exemplary embodiment, the manner of obtaining, based on the node-level attention information and the edge-level attention information corresponding to the key sub-sample object, the key local-level loss function is: determining, based on the node-level attention information corresponding to the key sub-sample object, the node-level loss function; determining, based on the edge-level attention information corresponding to the key sub-sample object, the edge-level loss function; and determining, based on the node-level loss function and the edge-level loss function, the key local-level loss function.

In any case, the key local-level loss function can be obtained. The objective of designing the key local-level loss function is: causing a sum of one or more attention weights corresponding to the one or more key sub-sample objects in the one or more sample objects meeting the reference condition at each angle to be close to 1 as much as possible, so that the interaction information prediction model learns more information about the one or more key sub-sample objects in the one or more sample objects meeting the reference condition during training. Because the one or more key sub-sample objects are one or more sub-sample objects performing interaction, the key local-level loss function can cause the interaction information prediction model to learn more information about the one or more sub-sample objects performing interaction during training.

Moreover, because the one or more key sub-sample objects are a part of all sub-sample objects included in the one or more sample objects meeting the reference condition, the key local-level loss function is a loss function paying attention to local information. The design of the key local-level loss function can add, by using one or more attention weights read by the one or more key sub-sample objects from the self-attention read function, a supervision signal used for paying attention to local information to the process of training the interaction information prediction model.

In an exemplary embodiment, when the type of the first sample object is a protein, and the type of the second sample object is a small molecule, because the entire small molecule usually interacts with partial amino acids in the protein, the one or more sample objects meeting the reference condition are the first sample object. The key sub-sample object in the first sample object refers to an amino acid in the protein used for bonding with the small molecule, the partial amino acids may be referred to as pocket amino acids, and information about the pocket amino acids can reflect pocket information of the protein.

Step 605. Reversely update, based on the global-level loss function and the key local-level loss function, a parameter of the initial interaction information prediction model.

After the global-level loss function is obtained based on step 602 and the key local-level loss function is obtained based on step 604, the parameter of the initial interaction information prediction model is reversely updated based on the global-level loss function and the key local-level loss function. Each time the parameter of the interaction information prediction model is updated, one time of training the interaction information prediction model is completed.

In a possible implementation, the process of reversely updating, based on the global-level loss function and the key local-level loss function, a parameter of the initial interaction information prediction model is: determining, based on the global-level loss function and the key local-level loss function, a comprehensive loss function, and reversely updating the parameter of the initial interaction information prediction model by using the comprehensive loss function. In a possible implementation, the manner of determining, based on the global-level loss function and the key local-level loss function, a comprehensive loss function is: performing weighted summation on the global-level loss function and the key local-level loss function, to obtain the comprehensive loss function. During the weighted summation, weights corresponding to the global-level loss function and the key local-level loss function are set according to experience or flexibly adjusted according to an application scenario. This is not limited in this embodiment of this application. Exemplarily, each weight corresponding to the global-level loss function and the key local-level loss function may be set to 1, and therefore the comprehensive loss function is a sum of the global-level loss function and the key local-level loss function.

In this embodiment of this application, the manner of reversely updating the parameter of the initial interaction information prediction model by using the comprehensive loss function is not limited. Exemplarily, the manner of reversely updating the parameter of the initial interaction information prediction model by using the comprehensive loss function is a gradient descent method.

In a possible implementation, the process of reversely updating, based on the global-level loss function and the key local-level loss function, a parameter of the initial interaction information prediction model may be immediately performed after a global-level loss function and a key local-level loss function are obtained using a group of sample objects, or may be performed after a small batch of global-level loss functions and a small batch of key local-level loss functions are obtained using a small batch of groups of sample objects. This is not limited in this embodiment of this application. The quantity of the small batch is set according to experience, or flexibly adjusted according to an application scenario. This is not limited in this embodiment of this application.

In a possible implementation, after the reversely updating, based on the global-level loss function and the key local-level loss function, a parameter of the initial interaction information prediction model, whether a parameter update process meets a termination condition is determined. When the parameter update process meets the termination condition, step 606 is performed. When the parameter update process does not meet the termination condition, step 601 to step 605 continue to be performed, until the parameter update process meets the termination condition, and step 606 is performed.

In an exemplary embodiment, that the parameter update process meets the termination condition includes but not limited to the following three cases:

Case A. A parameter update count reaches a count threshold.

The count threshold may be set according to experience, or flexibly adjusted according to an application scenario. This is not limited in this embodiment of this application.

Case B. The comprehensive loss function is less than a loss threshold.

The loss threshold may be set according to experience, or flexibly adjusted according to an application scenario. This is not limited in this embodiment of this application.

Case C. The comprehensive loss function converges.

That the comprehensive loss function converges means that as the parameter update count increases, fluctuation ranges of the comprehensive loss function fall within a reference range in update results of a reference count. For example, it is assumed that the reference range is from $-10^{-3}$ to $10^{-3}$, and it is assumed that the reference count is 10. If a fluctuation range of the comprehensive loss function in each of 10 parameter update results is from $-10^{-3}$ to $10^{-3}$, it is considered that the comprehensive loss function converges.

When any one of the foregoing three cases is met, it indicates that the parameter update process meets the termination condition, and step 606 is performed.

Step 606. Obtain, in response to a parameter update process meeting a termination condition, a target interaction information prediction model.

When the parameter update process meets the termination condition, the interaction information prediction model obtained when the parameter update process meets the termination condition is used as the trained target interaction information prediction model, thereby obtaining the target interaction information prediction model.

In a possible implementation, after the trained target interaction information prediction model is obtained, interaction information between two target objects is predicted using the target interaction information prediction model. Because the target interaction information prediction model is obtained after the global-level loss function and the key local-level loss function perform training, accuracy of the interaction information between the two target objects predicted using the target interaction information prediction model is relatively high.

In the embodiments of this application, in addition to the global-level loss function, the key local-level loss function is further designed. In the field of drug screening, the key local-level loss function can provide supervision for learning information about pocket amino acids in a protein. While paying attention to global information of the protein, this embodiment of this application can pay close attention to information blending at pocket amino acids, and can strengthen fusion learning of the global information and local information of the protein, thereby improving accuracy of protein-small molecule active information predicted by the trained drug screening model.

In an exemplary embodiment, the foregoing interaction information prediction model training method is performed before the embodiment shown in FIG. 2 is performed.

In this embodiment of this application, in the process of training the initial interaction information prediction model, the parameter of the initial interaction information prediction model is reversely updated based on the global-level loss function and the key local-level loss function. The global-level loss function can cause the model parameter update to pay attention to the global information; and the key local-level loss function can cause the model parameter update to pay attention to the local information. That is to say, in the embodiments of this application, the model training process not only pays attention to the global information, but also pays attention to the local information, to help improve the effect of training the interaction information prediction model, and then improve the accuracy of the interaction information predicted by using the trained target interaction information prediction model.

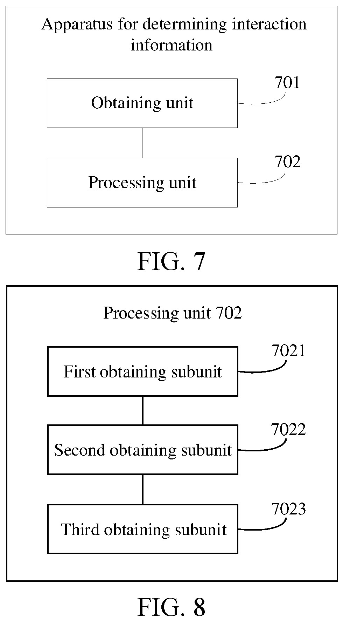

Referring to FIG. 7, an embodiment of this application provides an interaction information determining apparatus. The apparatus includes:

an obtaining unit 701, configured to obtain basic information of a first target object, basic information of a second target object, and a target interaction information prediction model, the target interaction information prediction model being trained by using a global-level loss function and a key local-level loss function, the key local-level loss function being determined based on attention information corresponding to one or more key sub-sample objects in one or more sample objects meeting a reference condition, and the one or more key sub-sample objects in the one or more sample objects meeting the reference condition being a part of all sub-sample objects of the one or more sample objects meeting the reference condition; and

a processing unit 702, configured to obtain target interaction information between the first target object and the second target object, by invoking the target interaction information prediction model to process the basic information of the first target object and the basic information of the second target object.

In a possible implementation, referring to FIG. 8, the processing unit 702 includes:

a first obtaining subunit 7021, configured to obtain, based on the basic information of the first target object, first basic feature information and first attention information corresponding to the first target object, by invoking the target interaction information prediction model; and obtain, based on the basic information of the second target object, second basic feature information and second attention information corresponding to the second target object;

a second obtaining subunit 7022, configured to obtain, based on the first basic feature information and the first attention information, first global feature information corresponding to the first target object; and obtain, based on the second basic feature information and the second attention information, second global feature information corresponding to the second target object; and

a third obtaining subunit 7023, configured to obtain, based on the first global feature information and the second global feature information, the target interaction information between the first target object and the second target object.

In a possible implementation, the first global feature information includes node-level first global feature information and edge-level first global feature information, the second global feature information includes node-level second global feature information and edge-level second global feature information, the target interaction information includes node-level target interaction information and edge-level target interaction information, and the target interaction information prediction model includes a first prediction processing model and a second prediction processing model; and the third obtaining subunit 7023 is configured to obtain node-level target interaction information between the first target object and the second target object by invoking the first prediction processing model to predict the node-level first global feature information and the node-level second global feature information; and obtain edge-level target interaction information between the first target object and the second target object, by invoking the second prediction processing model to predict the edge-level first global feature information and the edge-level second global feature information.

In a possible implementation, the basic information of the first target object is graph information of the first target object, and the target interaction information prediction model includes a first node message passing model and a first edge message passing model; and the first basic feature information includes node-level first basic feature information and edge-level first basic feature information; and the first obtaining subunit 7021 is further configured to obtain the node-level first basic feature information by invoking the first node message passing model to perform node-level feature extraction on the graph information of the first target object; and obtain the edge-level first basic feature information, by invoking the first edge message passing model to perform edge-level feature extraction on the graph information of the first target object.

In a possible implementation, the basic information of the second target object is graph information of the second target object; and the target interaction information prediction model includes a second node message passing model and a second edge message passing model, and the second basic feature information includes node-level second basic feature information and edge-level second basic feature information; and the first obtaining subunit 7021 is further configured to obtain the node-level second basic feature information, by invoking the second node message passing model to perform node-level feature extraction on the graph information of the second target object; and obtain the edge-level second basic feature information, invoking the second edge message passing model to perform edge-level feature extraction on the graph information of the second target object.

In a possible implementation, the first node message passing model includes a first node feature update layer and a first node-level feature output layer; and the first obtaining subunit 7021 is further configured to obtain a target node feature, by invoking the first node feature update layer to update a node feature in the graph information of the first target object; and obtain the node-level first basic feature information, by invoking the first node-level feature output layer to output the target node feature.

In a possible implementation, the first edge message passing model includes a first edge feature update layer and a first edge-level feature output layer; and the first obtaining subunit 7021 is further configured to obtain a target edge feature by invoking the first edge feature update layer to update an edge feature in the graph information of the first target object; and obtain the edge-level first basic feature information by invoking the first edge-level feature output layer to output the target edge feature.

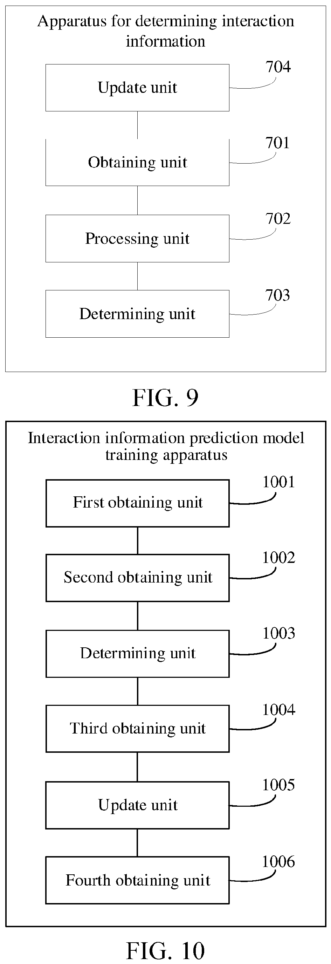

In a possible implementation, referring to FIG. 9, the apparatus further includes:

a determining unit 703, configured to determine, based on the first attention information corresponding to the first target object, a first key sub-target object in all sub-target objects of the first target object, the first key sub-target object being used for indicating a sub-target object that is in the first target object and that is used for interacting with the second target object; and determine, based on the second attention information corresponding to the second target object, a second key sub-target object in all sub-target objects of the second target object, the second key sub-target object being used for indicating a sub-target object that is in the second target object and that is used for interacting with the first target object.

In a possible implementation, the processing unit 702 is further configured to obtain attention information corresponding to a first sample object, attention information corresponding to a second sample object, and predictive interaction information between the first sample object and the second sample object by invoking an initial interaction information prediction model, the predictive interaction information being obtained based on global feature information corresponding to the first sample object and global feature information corresponding to the second sample object;

the obtaining unit 701 is further configured to obtain, based on the predictive interaction information and standard interaction information between the first sample object and the second sample object, a global-level loss function;

the determining unit 703 is further configured to determine, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object; and

the obtaining unit 701 is further configured to obtain, based on the attention information corresponding to the one or more key sub-sample objects in the one or more sample objects meeting the reference condition, a key local-level loss function.

referring to FIG. 9, the apparatus further includes:

an update unit 704, configured to reversely update, based on the global-level loss function and the key local-level loss function, a parameter of the initial interaction information prediction model; and

the obtaining unit 701 is further configured to obtain, in response to a parameter update process meeting a termination condition, a target interaction information prediction model.

In a possible implementation, the predictive interaction information includes node-level predictive interaction information and edge-level predictive interaction information; and the obtaining unit 701 is further configured to determine, based on the node-level predictive interaction information and the standard interaction information between the first sample object and the second sample object, a first loss sub-function; determine, based on the edge-level predictive interaction information and the standard interaction information between the first sample object and the second sample object, a second loss sub-function; determine, based on the node-level predictive interaction information and the edge-level predictive interaction information, a third loss sub-function; and determine, based on the first loss sub-function, the second loss sub-function, and the third loss sub-function, the global-level loss function.

In a possible implementation, the one or more sample objects meeting the reference condition are the first sample object and the second sample object; and the determining unit 703 is further configured to determine, in the attention information corresponding to the first sample object, attention information corresponding to a first key sub-sample object in the first sample object; and determine, in the attention information corresponding to the second sample object, attention information corresponding to a second key sub-sample object in the second sample object.

In a possible implementation, the obtaining unit 701 is further configured to determine, based on the attention information corresponding to the first key sub-sample object, a fourth loss sub-function; determine, based on the attention information corresponding to the second key sub-sample object, a fifth loss sub-function; and determine, based on the fourth loss sub-function and the fifth loss sub-function, the key local-level loss function.

In a possible implementation, a type of the first target object is a protein, and a type of the second target object is a small molecule; and the obtaining unit 701 is further configured to determine, based on structural information of the first target object, a spatial distance between amino acids in the first target object; determine, based on the spatial distance between the amino acids, an adjacency matrix corresponding to the first target object, the adjacency matrix being used for indicating an association between the amino acids in the first target object; obtain the graph information of the first target object according to the adjacency matrix corresponding to the first target object and the amino acids in the first target object, and use the graph information of the first target object as the basic information of the first target object; and obtain, based on atoms in the second target object and chemical bond information between the atoms, the graph information of the second target object, and use the graph information of the second target object as the basic information of the second target object.

In the embodiments of this application, the global-level loss function and the key local-level loss function are used for training the interaction information prediction model, and then the trained interaction information prediction model is used for determining the interaction information between the first target object and the second target object. The global-level loss function can cause the model training process to pay attention to the global information; and the key local-level loss function can cause the model training process to pay attention to the key local information. That is to say, in the embodiments of this application, the process of training the interaction information prediction model not only pays attention to the global information, but also pays attention to the key local information, so that the effect of training the interaction information prediction model is relatively good, and the accuracy of the interaction information between the first target object and the second target object determined by using the trained interaction information prediction model is relatively high.

Referring to FIG. 10, an embodiment of this application provides an interaction information prediction model training apparatus. The apparatus includes:

a first obtaining unit 1001, configured to invoke an initial interaction information prediction model, and obtain attention information corresponding to a first sample object, attention information corresponding to a second sample object, and predictive interaction information between the first sample object and the second sample object, the predictive interaction information being obtained based on global feature information corresponding to the first sample object and global feature information corresponding to the second sample object;

a second obtaining unit 1002, configured to obtain, based on the predictive interaction information and standard interaction information between the first sample object and the second sample object, a global-level loss function;

a determining unit 1003, configured to determine, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object; and

a third obtaining unit 1004, configured to obtain, based on the attention information corresponding to the one or more key sub-sample objects in the one or more sample objects meeting the reference condition, a key local-level loss function;

an update unit 1005, configured to reversely update, based on the global-level loss function and the key local-level loss function, a parameter of the initial interaction information prediction model; and

a fourth obtaining unit 1006, configured to obtain, in response to a parameter update process meeting a termination condition, a target interaction information prediction model.

In a possible implementation, the predictive interaction information includes node-level predictive interaction information and edge-level predictive interaction information; and the second obtaining unit 1002 is configured to determine, based on the node-level predictive interaction information and the standard interaction information between the first sample object and the second sample object, a first loss sub-function; determine, based on the edge-level predictive interaction information and the standard interaction information between the first sample object and the second sample object, a second loss sub-function; determine, based on the node-level predictive interaction information and the edge-level predictive interaction information, a third loss sub-function; and determine, based on the first loss sub-function, the second loss sub-function, and the third loss sub-function, the global-level loss function.

In a possible implementation, the one or more sample objects meeting the reference condition are the first sample object and the second sample object; and the determining unit 1003 is configured to determine, in the attention information corresponding to the first sample object, attention information corresponding to a first key sub-sample object in the first sample object; and determine, in the attention information corresponding to the second sample object, attention information corresponding to a second key sub-sample object in the second sample object.

In a possible implementation, the third obtaining unit 1004 is configured to determine, based on the attention information corresponding to the first key sub-sample object, a fourth loss sub-function; determine, based on the attention information corresponding to the second key sub-sample object, a fifth loss sub-function; and determine, based on the fourth loss sub-function and the fifth loss sub-function, the key local-level loss function.

In this embodiment of this application, in the process of training the initial interaction information prediction model, the parameter of the initial interaction information prediction model is reversely updated based on the global-level loss function and the key local-level loss function. The global-level loss function can cause the model parameter update to pay attention to the global information; and the key local-level loss function can cause the model parameter update to pay attention to the local information. That is to say, in the embodiments of this application, the model training process not only pays attention to the global information, but also pays attention to the local information, to help improve the effect of training the interaction information prediction model, and then improve the accuracy of the interaction information predicted by using the trained target interaction information prediction model.

When the apparatus provided in the foregoing embodiments implements functions of the apparatus, the division of the foregoing functional units is merely an example for description. In the practical application, the functions may be assigned to and completed by different functional units according to the requirements, that is, the internal structure of the device is divided into different functional units, to implement all or some of the functions described above. In addition, the apparatus and method embodiments provided in the foregoing embodiments belong to the same conception. For the specific implementation process, reference may be made to the method embodiments, and details are not described herein again.

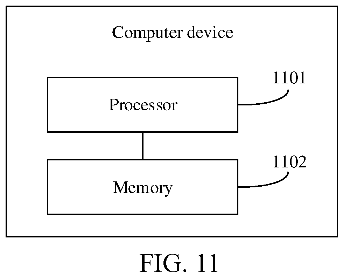

FIG. 11 is a schematic structural diagram of a computer device according to an embodiment of this application. The device may refer to a server. The server may vary greatly because a configuration or performance varies, and may include one or more central processing units (CPU) 1101 and one or more memories 1102. The one or more memories 1102 store at least one piece of program code, and the at least one piece of program code is loaded and executed by the one or more processors 1101 to implement the interaction information determining method or interaction information prediction model training method provided in the foregoing various method embodiments. Certainly, the server may further include components such as a wired or wireless network interface, a keyboard, and an input/output interface, to facilitate inputs/outputs. The server may further include another component configured to implement functions of a device. Details are not described herein again.

In an exemplary embodiment, a computer device is further provided, the computer device including a processor and a memory, the memory storing at least one piece of program code. The at least one piece of program code is loaded and executed by one or more processors to cause the computer device to implement the interaction information determining method or the interaction information prediction model training method described above.

In an exemplary embodiment, a non-transitory computer-readable storage medium is further provided, the non-transitory computer-readable storage medium storing at least one piece of program code, the at least one piece of program code being loaded and executed by a processor of a computer device to cause the computer to implement the interaction information determining method or the interaction information prediction model training method described above.

In a possible implementation, the non-transitory computer-readable storage medium may be a read-only memory (ROM), a random access memory (random-access memory, RAM), a compact disc read-only memory (CD-ROM), a magnetic tape, a floppy disk, an optical data storage device, and the like.

In an exemplary embodiment, a computer program product or a computer program is further provided. The computer program product or the computer program includes computer instructions, and the computer instructions are stored in a computer-readable storage medium. A processor of a computer device reads the computer instructions from the computer-readable storage medium, and the processor executes the computer instructions to cause the computer device to implement any interaction information determining method or interaction information prediction model training method described above.

In the specification and claims of this application, the terms "first", "second", and so on are intended to distinguish between similar objects but do not necessarily describe a specific order or sequence. It is to be understood that data used in this way is interchangeable in a suitable case, so that the embodiments of this application described herein can be implemented in a sequence in addition to the sequence shown or described herein. The foregoing implementations described in the following exemplary embodiments do not represent all implementations that are consistent with this application. Instead, they are merely examples of apparatuses and methods consistent with aspects related to this application as recited in the appended claims.

It is to be understood that "plurality of" mentioned in this specification means two or more. The term "and/or" describes an association relationship for describing associated objects and represents that three relationships may exist. For example, A and/or B may represent the following three cases: Only A exists, both A and B exist, and only B exists. The character "/" generally indicates an "or" relationship between the associated objects.

The foregoing descriptions are merely exemplary embodiments of this application, but are not intended to limit this application. Any modification, equivalent replacement, or improvement made within the spirit and principle of this application shall fall within the protection scope of this application.

**Claims**

1. A method for determining interaction information, performed by a computer device, the method comprising:

   obtaining basic information of a first target object, basic information of a second target object, and a target interaction information prediction model, the first target object and the second target object each comprising a protein or a small molecule of a drug respectively, the target interaction information prediction model being trained by using a global-level loss function and a key local-level loss function, the key local-level loss function being determined based on attention information corresponding to one or more key sub-sample objects in one or more sample objects meeting a reference condition, and the one or more key sub-sample objects in the one or more sample objects meeting the reference condition being a part of all sub-sample objects of the one or more sample objects meeting the reference condition, one key sub-sample object in one sample object being a sub-sample object interacting with another sample object, one sample object meeting the reference condition indicating the one sample object comprises at least one key sub-sample object; and
   obtaining target interaction information for drug screening between the first target object and the second target object, by invoking the target interaction information prediction model to process the basic information of the first target object and the basic information of the second target object.

2. The method according to claim 1, wherein the obtaining target interaction information for drug screening between the first target object and the second target object, by invoking the target interaction information prediction model to process the basic information of the first target object and the basic information of the second target object comprises:

   obtaining, based on the basic information of the first target object, first basic feature information and first attention information corresponding to the first target object, by invoking the target interaction information prediction model; and obtaining, based on the basic information of the second target object, second basic feature information and second attention information corresponding to the second target object, by invoking the target interaction information prediction model;
   obtaining, based on the first basic feature information and the first attention information, first global feature information corresponding to the first target object; and obtaining, based on the second basic feature information and the second attention information, second global feature information corresponding to the second target object; and
   obtaining, based on the first global feature information and the second global feature information, the target interaction information between the first target object and the second target object.

3. The method according to claim 2, wherein the first global feature information comprises node-level first global feature information and edge-level first global feature information, the second global feature information comprises node-level second global feature information and edge-level second global feature information, the target interaction information comprises node-level target interaction information and edge-level target interaction information, and the target interaction information prediction model comprises a first prediction processing model and a second prediction processing model; and

the obtaining, based on the first global feature information and the second global feature information, the target interaction information between the first target object and the second target object comprises:

obtaining node-level target interaction information between the first target object and the second target object, by invoking the first prediction processing model to predict the node-level first global feature information and the node-level second global feature information; and

obtaining edge-level target interaction information between the first target object and the second target object, by invoking the second prediction processing model to predict the edge-level first global feature information and the edge-level second global feature information.

4. The method according to claim 2, wherein the basic information of the first target object is graph information of the first target object, and the target interaction information prediction model comprises a first node message passing model and a first edge message passing model; and the first basic feature information comprises node-level first basic feature information and edge-level first basic feature information; and

the obtaining, based on the basic information of the first target object, first basic feature information corresponding to the first target object by the invoking the target interaction information prediction model comprises:

obtaining the node-level first basic feature information, by invoking the first node message passing model to perform node-level feature extraction on the graph information of the first target object; and

obtaining the edge-level first basic feature information, by invoking the first edge message passing model to perform edge-level feature extraction on the graph information of the first target object.

5. The method according to claim 2, wherein the basic information of the second target object is graph information of the second target object; and the target interaction information prediction model comprises a second node message passing model and a second edge message passing model, and the second basic feature information comprises node-level second basic feature information and edge-level second basic feature information; and

the obtaining, based on the basic information of the second target object, second basic feature information corresponding to the second target object, by invoking the target interaction information prediction model comprises:

obtaining the node-level second basic feature information, by invoking the second node message passing model to perform node-level feature extraction on the graph information of the second target object; and

obtaining the edge-level second basic feature information, by invoking the second edge message passing model to perform edge-level feature extraction on the graph information of the second target object.

6. The method according to claim 4, wherein the first node message passing model comprises a first node feature update layer and a first node-level feature output layer; and the obtaining the node-level first basic feature information, by invoking the first node message passing model to perform node-level feature extraction on the graph information of the first target object comprises:

obtaining a target node feature, by invoking the first node feature update layer to update a node feature in the graph information of the first target object; and

obtaining the node-level first basic feature information, by invoking the first node-level feature output layer to output the target node feature.

7. The method according to claim 4, wherein the first edge message passing model comprises a first edge feature update layer and a first edge-level feature output layer; and the obtaining the edge-level first basic feature information, by invoking the first edge message passing model to perform edge-level feature extraction on the graph information of the first target object comprises:

obtaining a target edge feature, by invoking the first edge feature update layer to update an edge feature in the graph information of the first target object; and

obtaining the edge-level first basic feature information, by invoking the first edge-level feature output layer to output the target edge feature.

8. The method according to claim 2, wherein after the obtaining, based on the basic information of the first target object, first basic feature information and first attention information corresponding to the first target object; and obtaining, based on the basic information of the second target object, second basic feature information and second attention information corresponding to the second target object, the method further comprises:

determining, based on the first attention information corresponding to the first target object, a first key sub-target object in all sub-target objects of the first target object, the first key sub-target object being used for indicating a sub-target object that is in the first target object and that is used for interacting with the second target object; and
determining, based on the second attention information corresponding to the second target object, a second key sub-target object in all sub-target objects of the second target object, the second key sub-target object being used for indicating a sub-target object that is in the second target object and that is used for interacting with the first target object.

9. The method according to any one of claims 1 to 8, wherein a type of the first target object is a protein, and a type of the second target object is a small molecule; and the obtaining basic information of a first target object and basic information of a second target object comprises:

determining, based on structural information of the first target object, a spatial distance between amino acids in the first target object;
determining, based on the spatial distance between the amino acids, an adjacency matrix corresponding to the first target object, the adjacency matrix being used for indicating an association between the amino acids in the first target object;
obtaining the graph information of the first target object according to the adjacency matrix corresponding to the first target object and the amino acids in the first target object, and using the graph information of the first target object as the basic information of the first target object; and
obtaining, based on atoms in the second target object and chemical bond information between the atoms, the graph information of the second target object, and using the graph information of the second target object as the basic information of the second target object.

10. An interaction information prediction model training method, performed by a computer device, the method comprising:

obtaining attention information corresponding to a first sample object, attention information corresponding to a second sample object, and predictive interaction information between the first sample object and the second sample object by invoking an initial interaction information prediction model, the predictive interaction information being obtained based on global feature information corresponding to the first sample object and global feature information corresponding to the second sample object;
obtaining, based on the predictive interaction information and standard interaction information between the first sample object and the second sample object, a global-level loss function;
determining, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object;
obtaining, based on the attention information corresponding to the one or more key sub-sample objects in the one or more sample objects meeting the reference condition, a key local-level loss function;
reversely updating, based on the global-level loss function and the key local-level loss function, a parameter of the initial interaction information prediction model; and
obtaining, in response to a parameter update process meeting a termination condition, a target interaction information prediction model.

11. The method according to claim 10, wherein the predictive interaction information comprises node-level predictive interaction information and edge-level predictive interaction information; and the obtaining, based on the predictive interaction information and standard interaction information between the first sample object and the second sample object, a global-level loss function comprises:

determining, based on the node-level predictive interaction information and the standard interaction information between the first sample object and the second sample object, a first loss sub-function;

determining, based on the edge-level predictive interaction information and the standard interaction information between the first sample object and the second sample object, a second loss sub-function;

determining, based on the node-level predictive interaction information and the edge-level predictive interaction information, a third loss sub-function; and

determining, based on the first loss sub-function, the second loss sub-function, and the third loss sub-function, the global-level loss function.

12. The method according to claim 10 or 11, wherein the one or more sample objects meeting the reference condition are the first sample object and the second sample object; and

the determining, in attention information corresponding to one or more sample objects meeting a reference condition, attention information corresponding to one or more key sub-sample objects in the one or more sample objects meeting the reference condition comprises:

determining, in the attention information corresponding to the first sample object, attention information corresponding to a first key sub-sample object in the first sample object; and

determining, in the attention information corresponding to the second sample object, attention information corresponding to a second key sub-sample object in the second sample object.

13. The method according to claim 12, wherein the obtaining, based on the attention information corresponding to the one or more key sub-sample objects in the one or more sample objects meeting the reference condition, a key local-level loss function comprises:

determining, based on the attention information corresponding to the first key sub-sample object, a fourth loss sub-function;

determining, based on the attention information corresponding to the second key sub-sample object, a fifth loss sub-function; and

determining, based on the fourth loss sub-function and the fifth loss sub-function, the key local-level loss function.

14. An interaction information determining apparatus, comprising:

an obtaining unit, configured to obtain basic information of a first target object, basic information of a second target object, and a target interaction information prediction model, the target interaction information prediction model being obtained through training by using a global-level loss function and a key local-level loss function, the key local-level loss function being determined based on attention information corresponding to one or more key sub-sample objects in one or more sample objects meeting a reference condition, and the one or more key sub-sample objects in the one or more sample objects meeting the reference condition being a part of all sub-sample objects of the one or more sample objects meeting the reference condition; and

a processing unit, configured to invoke the target interaction information prediction model to process the basic information of the first target object and the basic information of the second target object, and obtain target interaction information between the first target object and the second target object.

15. An interaction information prediction model training apparatus, comprising:

a first obtaining unit, configured to invoke an initial interaction information prediction model, and obtain attention information corresponding to a first sample object, attention information corresponding to a second sample object, and predictive interaction information between the first sample object and the second sample object, the predictive interaction information being obtained based on global feature information corresponding to the first sample object and global feature information corresponding to the second sample object;

a second obtaining unit, configured to obtain, based on the predictive interaction information and standard interaction information between the first sample object and the second sample object, a global-level loss function;

a determining unit, configured to determine, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object;

a third obtaining unit, configured to obtain, based on the attention information corresponding to the one or more key sub-sample objects in the one or more sample objects meeting the reference condition, a key local-level loss function;

an update unit, configured to reversely update, based on the global-level loss function and the key local-level

**EP 4 170 662 A1**

loss function, a parameter of the initial interaction information prediction model; and

a fourth obtaining unit, configured to obtain, in response to a parameter update process meeting a termination condition, a target interaction information prediction model.

16. A computer device, comprising a processor and a memory, the memory storing at least one piece of program code, the at least one piece of program code being loaded and executed by the processor to cause the computer device to implement the method according to any one of claims 1 to 9, or implement the interaction information prediction model training method according to any one of claims 10 to 13.

17. A non-transitory computer-readable storage medium, storing at least one piece of program code, the at least one piece of program code being loaded and executed by a processor to cause a computer to implement the method according to any one of claims 1 to 9, or implement the interaction information prediction model training method according to any one of claims 10 to 13.

18. A computer program product, comprising computer instructions, the computer instructions being stored in a computer-readable storage medium, a processor of a computer device reading the computer instructions from the computer-readable storage medium, and the processor executing the computer instructions to cause the computer device to implement the method according to any one of claims 1 to 9, or implement the interaction information prediction model training method according to any one of claims 10 to 13.

FIG. 1

| Obtain basic information of a first target object, basic information of a second target object, and a target interaction information prediction model, the first target object and the second target object each comprising a protein or a small molecule of a drug respectively, the target interaction information prediction model being trained by using a global-level loss function and a key local-level loss function | 201 |
|---|---|

| Obtain target interaction information for drug screening between the first target object and the second target object, by invoking the target interaction information prediction model to process the basic information of the first target object and the basic information of the second target object | 202 |
|---|---|

FIG. 2

FIG. 3

Obtain, based on the basic information of the first target object, first basic feature information and first attention information corresponding to the first target object, by invoking the target interaction information prediction model; and obtain, based on the basic information of the second target object, second basic feature information and second attention information corresponding to the second target object, by invoking the target interaction information prediction model

2021

Obtain first global feature information corresponding to the first target object; and obtain, based on the second basic feature information and the second attention information, second global feature information corresponding to the second target object based on the first basic feature information and the first attention information

2022

Obtain the target interaction information between the first target object and the second target object based on the first global feature information and the second global feature information

2023

## FIG. 4

## FIG. 5

Invoke an initial interaction information prediction model, and obtain attention information corresponding to a first sample object, attention information corresponding to a second sample object, and predictive interaction information between the first sample object and the second sample object 601

Obtain, based on the predictive interaction information and standard interaction information between the first sample object and the second sample object, a global-level loss function 602

Determine, in attention information corresponding to one or more sample objects meeting a reference condition, attention information corresponding to one or more key sub-sample objects in the one or more sample objects meeting the reference condition, the one or more sample objects meeting the reference condition being at least one of the first sample object and the second sample object 603

Obtain, based on the attention information corresponding to the one or more key sub-sample objects in the one or more sample objects meeting the reference condition, a key local-level loss function 604

Reversely update, based on the global-level loss function and the key local-level loss function, a parameter of the initial interaction information prediction model 605

Obtain, in response to a parameter update process meeting a termination condition, a target interaction information prediction model 606

FIG. 6

Apparatus for determining interaction information

Obtaining unit — 701

Processing unit — 702

**FIG. 7**

Processing unit 702

First obtaining subunit — 7021

Second obtaining subunit — 7022

Third obtaining subunit — 7023

**FIG. 8**

Apparatus for determining interaction
information

| Update unit | 704 |

| Obtaining unit | 701 |

| Processing unit | 702 |

| Determining unit | 703 |

## FIG. 9

Interaction information prediction model
training apparatus

| First obtaining unit | 1001 |

| Second obtaining unit | 1002 |

| Determining unit | 1003 |

| Third obtaining unit | 1004 |

| Update unit | 1005 |

| Fourth obtaining unit | 1006 |

## FIG. 10

FIG. 11

## INTERNATIONAL SEARCH REPORT

International application No.

**PCT/CN2021/119651**

**A. CLASSIFICATION OF SUBJECT MATTER**

G16C 20/50(2019.01)i

According to International Patent Classification (IPC) or to both national classification and IPC

**B. FIELDS SEARCHED**

Minimum documentation searched (classification system followed by classification symbols)

G16C

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 全局, 局部, 相互作用, 蛋白质, 小分子, 预测, 损失函数, 训练, 注意力, 模型, global, local, interaction, protein, small molecule, predict, loss function, training, attention, model

**C. DOCUMENTS CONSIDERED TO BE RELEVANT**

| Category* | Citation of document, with indication, where appropriate, of the relevant passages | Relevant to claim No. |
|---|---|---|
| PX | CN 112151128 A (TENCENT TECHNOLOGY SHENZHEN CO., LTD.) 29 December 2020 (2020-12-29)<br>claims 1-12, description paragraphs [0004]-[0326] | 1-18 |
| A | CN 111613273 A (INSTITUTE OF ANIMAL HUSBANDRY AND VETERINARY MEDICINE, ANHUI ACADEMY OF AGRICULTURAL SCIENCES) 01 September 2020 (2020-09-01)<br>description, paragraphs [0043]-[0077] | 1-18 |
| A | CN 111667884 A (TIANJIN UNIVERSITY) 15 September 2020 (2020-09-15)<br>entire document | 1-18 |
| A | CN 110415763 A (TENCENT TECHNOLOGY SHENZHEN CO., LTD. et al.) 05 November 2019 (2019-11-05)<br>entire document | 1-18 |
| A | CN 107742061 A (SUN YAT-SEN UNIVERSITY) 27 February 2018 (2018-02-27)<br>entire document | 1-18 |
| A | WO 2017174580 A1 (BOEHRINGER INGELHEIM RCV GMBH & CO., KG. et al.) 12 October 2017 (2017-10-12)<br>entire document | 1-18 |

☐ Further documents are listed in the continuation of Box C.　　☑ See patent family annex.

| | |
|---|---|
| *　Special categories of cited documents:<br>"A"　document defining the general state of the art which is not considered to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search | Date of mailing of the international search report |
|---|---|
| **09 December 2021** | **21 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/119651**

| Patent document cited in search report | | | Publication date (day/month/year) | Patent family member(s) | | | Publication date (day/month/year) |
|---|---|---|---|---|---|---|---|
| CN | 112151128 | A | 29 December 2020 | None | | | |
| CN | 111613273 | A | 01 September 2020 | None | | | |
| CN | 111667884 | A | 15 September 2020 | None | | | |
| CN | 110415763 | A | 05 November 2019 | None | | | |
| CN | 107742061 | A | 27 February 2018 | None | | | |
| WO | 2017174580 | A1 | 12 October 2017 | KR | 20180134942 | A | 19 December 2018 |
| | | | | EP | 3440518 | A1 | 13 February 2019 |
| | | | | CN | 109313419 | A | 05 February 2019 |
| | | | | JP | 2019522802 | A | 15 August 2019 |
| | | | | US | 2021149361 | A1 | 20 May 2021 |

Form PCT/ISA/210 (patent family annex) (January 2015)

**REFERENCES CITED IN THE DESCRIPTION**

**Patent documents cited in the description**

- CN 202011112368